# EUROPEAN PATENT APPLICATION

(11) **EP 4 748 328 A1**
(43) Date of publication of application: **27.05.2026**
(21) Application number: 23945838.3
(22) Date of filing: 18.07.2023
(51) Int. Cl.: A61B 17/92, A61B 17/86

(54) **TREATMENT INSTRUMENT**

(71) Applicant: Spine Chronicle Japan Co., Ltd., Kanazawa-city, Ishikawa 920-0848 (JP)
(72) Inventor: YONEZAWA, Noritaka, Kanazawa-city, Ishikawa 920-0848 (JP)
(74) Representative: Plougmann Vingtoft a/s
(86) International application number: PCT/JP2023/026284
(87) International publication number: WO 2025/017834

(57) **Abstract**

Provided is a treatment device including a bone cement molding tool and a bone cement fixation tool, which are inserted into an internal fixation device that supports and internally fixes bone cement filled into a vertebral body from a pedicle, and is configured as follows. The internal fixation device has a through hole in the direction of the central axis from a head to the tip and can be inserted into and pulled out from bone cement along a central axis thereof. After the internal fixation device is screwed into the bone cement, the bone cement molding tool is inserted into the bone cement through the through hole in the internal fixation device to mold hole in a predetermined shape. The bone cement fixation tool has a tip section to be inserted into the hole molded above, and the tip section is inserted into the hole through a through hole in the internal fixation device, to inhibit rotation and/or movement in the direction of pulling-out of the bone cement.

## Description

### Technical Field

The present invention relates to a treatment device for operating a cylindrical internal fixation device for medical use, and is particularly suitable for internal fixation surgery of an affected vertebral body.

### Background Art

Vertebroplasty and spinal fusion are known treatments for compression fractures of the spine. The spine consists of stacked vertebral bodies and their supporting vertebral arches, pedicles, and articular processes. The vertebral body is a cylindrical bone which is supported by two pedicles on either side extending from the vertebral arch. The vertebral bodies sandwich the nucleus pulposus and the annulus fibrosus surrounds it. The vertebral arches connect vertically via the upper and lower articular processes and support the vertebral body via the pedicles, forming the spine. A compression fracture is a condition in which the vertebral body is crushed and damaged by vertical compression. Vertebroplasty is a surgical procedure to reconstruct the crushed vertebral body, for example, by filling the vertebra with medical cement. Spinal fusion is a technique to fix the crushed vertebral body to undamaged vertebral bodies above and below the crushed one using instruments. Screws are threaded into the crushed vertebral body and uncrushed vertebral bodies above and below the crushed one from the pedicles; and heads of the screws are fixed to each other via a vertical rod connecting them. Vertebroplasty is employed when the conditions are mild, but spinal fusion is required when symptoms are severe.

Various screws have been proposed for use in these treatments.

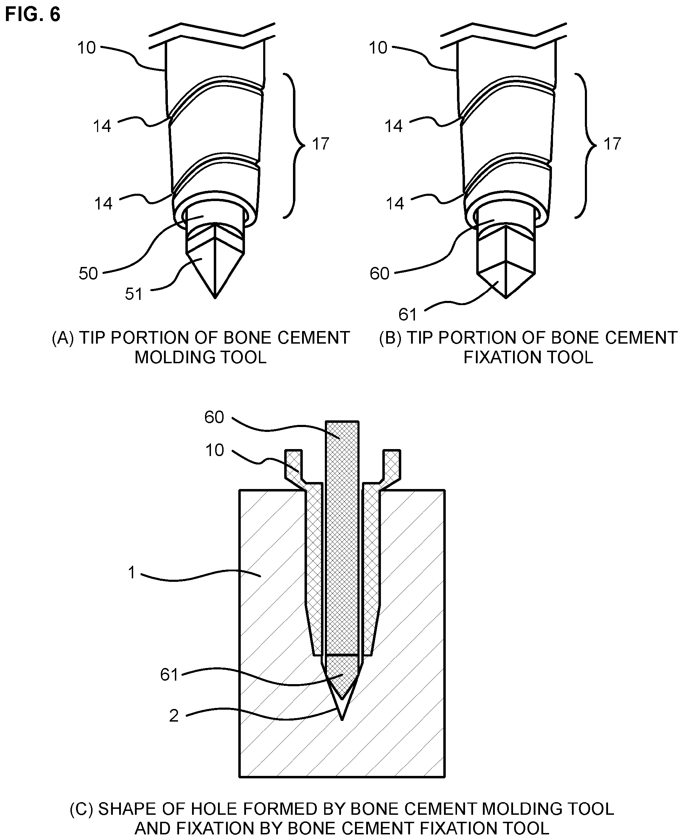

PTL 1 discloses a medical screw provided with a longitudinal through hole along its central axis and a lateral opening communicating with the through hole. An injection instrument such as a syringe is attached to a head of the screw, and bone cement and/or biologically active substances is injected into surrounding bones through the through hole on the side surface to reinforce the surrounding bones ([0053] - [0057], FIGS. 6-8).

PTL 2 discloses a medical screw that is said to be easily removed from a fracture site it is threaded in. The disclosed is a medical screw with a hollow section extending from a head to a tip along a central axis. The screw has a threaded side surface and is screwed into a bone. The screw is provided with a reverse thread (i.e., a reverse internal thread) near an inside tip of the hollow section, and can be pulled out by using a pulling-out operation jig equipped with a tip section that engages with the reverse internal thread. More specifically, the pulling-out operation jig is inserted through the hollow section, engaging the tip section with the reverse internal thread of the screw for integration, and the pulling-out operation jig is rotated to remove the screw out of the body.

### CITATION LIST

### Patent Document

PTL 1: U.S. Patent Application Publication 2011/0040337A1
PTL 2: Japanese Patent Application Laid-Open No. 2016-209295

### SUMMARY OF THE INVENTION

### Problems to be Solved by the Invention

The present inventor found the problem of insufficient development of medical devices and screws suitable for the combined vertebroplasty and pedicle formation as described above.

The inventor invented a treatment device to solve this problem, and filed an international application as PCT/JP2022/036147. The applied invention is a treatment device that is integrated with a screw having a through hole along a central axis and threads that can be tapped on a side surface thereof. The device is configured such that bone cement can be injected into the affected area through the through hole of the screw with a screwdriver connected to a head of the screw, and then the screw can be screwed into the bone cement. Since the screwdriver can be operated without the need to reattach the screwdriver after the injection of the bone cement, the screw can be screwed quickly before the bone cement hardens.

Conventional screws as in PTL 1, on the contrary, are not designed to be screwed into pre-injected bone cement; cement and/or other materials is injected around the screw through an opening in a side wall to reinforce the surrounding bone after the screw is screwed in. Therefore, it is essentially bone that engages with the screw threads, and it is believed that the screw can be pulled out relatively easily by reversing the screw. If pulling out is still expected to be difficult, a screw and a pulling-out operation jig that is designed for pulling out is used, as described in PTL 2.

The inventor has examined more deeply the risk about the danger that the screw would be too tightly engaged in the bone cement and could not be successfully removed, and has found that such danger is manifested when the bond due to the screw's engagement with the bone cement is tighter than the bond between the bone cement and the surrounding bone and other body tissue. The inventor has noticed that in that case using a jig for pulling out as described in PTL 2 does not solve the problem. Although a jig for pulling out as described in PTL 2 can strongly connect the screw to a screwdriver and can transmit strong force to the screwdriver, which aids in pulling out when the surrounding area is body tissue such as the patient's bone, if the bond between the bone cement and the screw is stronger than the bond between the bone cement and the surrounding bone or other body tissue, the bone cement might separate from the surrounding body tissue instead of the screw detaching from the bone cement, which will not aid in removal.

To solve this problem, the inventor invented an easy-to-remove internal fixation device and filed an international application as PCT/JP2023/022593. The internal fixation device of the present invention is an alternative to the screw described above, in which the portion inserted into the bone cement has grooves instead of threads. The threads push the surrounding bone cement apart as they are inserted into the bone cement, so there is no gap, whereas the grooves only allow bone cement to enter due to its viscosity, so if a gap is created or even if no gap is created, the adhesion with the bone cement is weak. Therefore, pulling out requires only small force.

The term "internal fixation device" used herein refers to an instrument to be inserted from a pedicle into bone cement within a vertebral body to support and internally fix the bone cement within the vertebral body from the pedicle side, and is redefined as an instrument including the screw described above.

The inventor has recognized another issue. When inserting the internal fixation device into bone cement, the device is generally screwed using the threads for tapping (i.e., thread provided at least on the head side for screwing into the pedicle), while when removing it, the internal fixation device is rotated in the opposite direction to when it is screwed in. However, if the bond between the internal fixation device and the bone cement is stronger than the fusion between the bone cement and the surrounding bone or other affected tissue, the bone cement cannot be rotated and removed.

An object of the present invention is to provide a treatment device that assists in the removal of an internal fixation device that is inserted through a pedicle into bone cement pre-filled into the vertebral body.

Means for solving these problems will be described below, but other issues and novel features will become apparent from the description herein and the accompanying drawings.

### Means for Solving Problems

According to one embodiment of the present invention, a means for solving the problems is as follows.

That is, a treatment device includes a bone cement molding tool and a bone cement fixation tool, which are inserted into an internal fixation device that supports and internally fixes bone cement filled into a vertebral body from a pedicle, and is configured as follows. The internal fixation device has a through hole in the direction of the central axis from a head to the tip and can be inserted into and pulled out from bone cement along a central axis thereof. The bone cement molding tool is inserted into the bone cement through the through hole in the internal fixation device to mold a hole in a predetermined shape. The bone cement fixation tool has a tip section to be inserted into the hole molded above, and the tip section is inserted into the hole through a through hole in the internal fixation device, to inhibit rotation and/or movement in the direction of pulling-out of the bone cement.

### Effect of the Invention

The effect to be produced by the above-described embodiment will be briefly described below.

That is, a treatment device that assists in the removal of an internal fixation device that is inserted through a pedicle into bone cement pre-filled into the vertebral body can be provided.

### BRIEF DESCRIPTION OF THE DRAWINGS

FIG. 1 is an explanatory drawing schematically illustrating a cross-sectional structure to show an example configuration of a treatment device according to the present invention, including a bone cement molding tool.
FIG. 2 is an explanatory drawing schematically illustrating a cross-sectional structure to show an example configuration of a treatment device according to the present invention, including a bone cement fixation tool.
FIG. 3 is an explanatory drawing illustrating the bottoms of tip sections (quadrilateral) viewed from the proximal end to show a first example configuration of the bone cement molding tool and the bone cement fixation tool.
FIG. 4 is an explanatory drawing illustrating the bottoms of tip sections (star-shape) viewed from the proximal end to show a third example configuration of the bone cement molding tool and the bone cement fixation tool.
FIG. 5 is an explanatory drawing illustrating the overviewed appearance and cross-sectional structure of the tip portions to show a first example configuration of the bone cement molding tool and the bone cement fixation tool.
FIG. 6 is an explanatory drawing illustrating the overviewed bottoms and cross-sectional structure of the tip sections of the bone cement molding tool and the bone cement fixation tool to show a second example configuration thereof.
FIG. 7 is an explanatory drawing overviewing an example use of the internal fixation device according to the present invention.
FIG. 8 is a top view, a front view and a bottom view of an example configuration of an internal fixation device according to the present invention.
FIG. 9 is an explanatory drawing schematically illustrating a cross-sectional structure to show an example configuration of a treatment device according to the present invention.
FIG. 10 is an explanatory drawing schematically illustrating the first half of a treatment procedure of implanting an internal fixation device in treatment using the treatment device according to the present invention.
FIG. 11 is an explanatory drawing schematically illustrating an example of the second half of the treatment procedure of implanting the internal fixation device in treatment using the treatment device according to the present invention.
FIG. 12 is an explanatory drawing schematically illustrating another example of the second half of the treatment procedure of implanting the internal fixation device in treatment using the treatment device according to the present invention.
FIG. 13 is an explanatory drawing schematically illustrating an example of a procedure of removing the internal fixation device in treatment using the treatment device according to the present invention.
FIG. 14 is an explanatory drawing schematically illustrating another example of the procedure of removing the internal fixation device in treatment using the treatment device according to the present invention.
FIG. 15 is an explanatory drawing illustrating the bottoms of tip sections (cross-shape) viewed from the proximal end to show a fourth example configuration of the bone cement molding tool and the bone cement fixation tool.
FIG. 16 is an explanatory drawing illustrating the overviewed appearance and cross-sectional structure of the tip sections to show a fifth example configuration of the bone cement molding tool and the bone cement fixation tool.
FIG. 17 is an explanatory drawing illustrating the overviewed appearance and cross-sectional structure of the tip sections to show a sixth example configuration of the bone cement molding tool and the bone cement fixation tool.
FIG. 18 is an explanatory drawing schematically illustrating an example of the procedure of removing the internal fixation device in treatment using the treatment device of a third embodiment according to the present invention.
FIG. 19 is an explanatory drawing schematically illustrating another example of the procedure of removing the internal fixation device in treatment using the treatment device of a third embodiment according to the present invention.
FIG. 20 is an explanatory drawing schematically illustrating example configurations of a bone cement molding tool and a screwdriver according to the treatment device of fourth and fifth embodiments of the present invention.
FIG. 21 is an explanatory drawing schematically illustrating the second half of the treatment procedure of implanting the internal fixation device in treatment using the treatment device according to the fifth embodiment.
FIG. 22 is an explanatory drawing schematically illustrating an example of the procedure of removing the internal fixation device in treatment using the treatment device of a sixth embodiment according to the present invention.

### DETAILED DESCRIPTION OF THE INVENTION

### 1. Outline of Embodiment

First, an outline of a representative embodiment disclosed in the present application will be given. Reference signs in the drawings that are referred to in parentheses in the outline description of representative embodiment are merely illustrative of what is included in the concept of the components to which they refer.

The term "proximal" herein is a medical term referring to the side closer to, and "distal" refers to the side farther away the centerline of a patient's body. Bone cement is medical cement composed mainly of, for example, calcium phosphate and polymethylmethacrylate, and hardens over time.

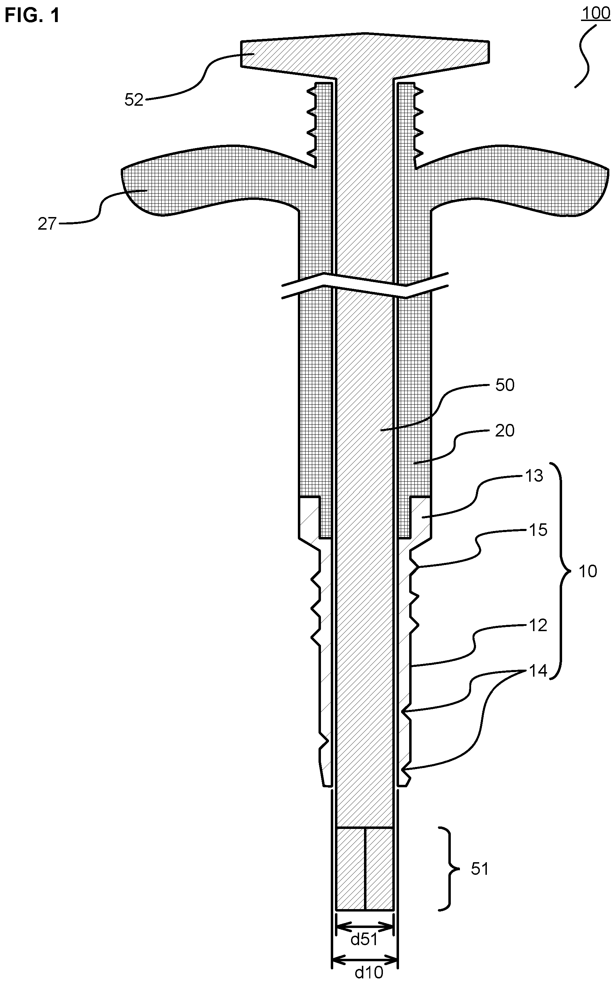

### [1] Pulling out support tool that inhibit rotation/pulling-out of bone cement (FIGS. 1 - 5, FIGS. 15 - 17, and FIG. 20)

A representative embodiment disclosed in this application is treatment device (100) that is inserted into internal fixation device (10) that supports and internally fixes bone cement filled into a vertebral body from a pedicle, treatment device (100) being provided with bone cement molding tool (50) and bone cement fixation tool (60), and is configured as follows.

The internal fixation device has through hole (16) in the direction of the central axis from head (13) to the tip and can be inserted into and pulled out of bone cement (1) along the central axis.

The bone cement molding tool is inserted into the bone cement through the through hole in the internal fixation device to mold hole (2) in a predetermined shape.

The bone cement fixation tool has tip section (61) to be inserted into the hole and is inserted into bone cement (1) in which the hole has been molded through the through hole in the internal fixation device to inhibit rotation around the central axis and/or movement in the pull-out direction of the bone cement.

This provides a treatment device that assists in the removal of an internal fixation device that is inserted through a pedicle into bone cement pre-filled into the vertebral body and supports and internally fixes the bone cement.

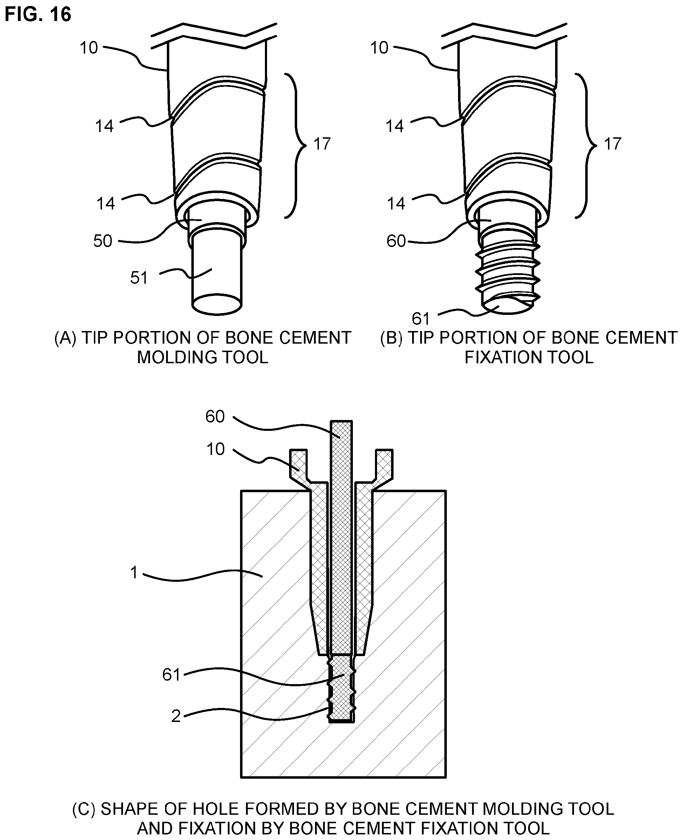

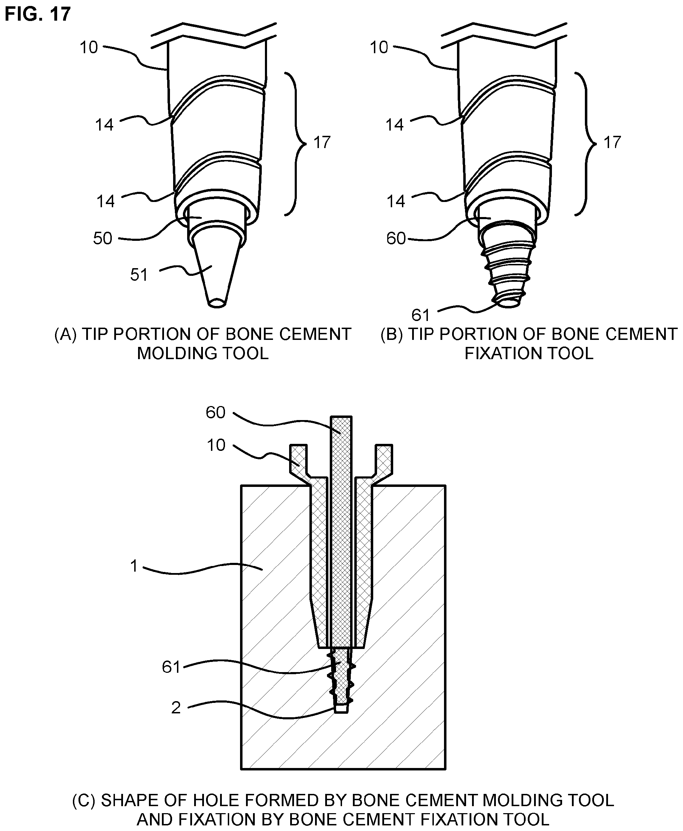

### [2] Threads for tapping of bone cement fixation tool are screwed into circular hole and fixed (FIG. 16 and FIG. 17).

In the treatment device of [1], the tip section of the bone cement molding tool is round column (FIG. 16) or round column tapered with a decreasing diameter toward the tip (FIG. 17). Hole (2) which is cylindrical or round column tapered in shape with a decreasing diameter in the depth direction is molded. The bone cement fixation tool has threads that tap a side wall of the hole and screwed thereinto.

This allows the bone cement fixation tool to inhibit movement of the bone cement in both the rotational and pulling out directions.

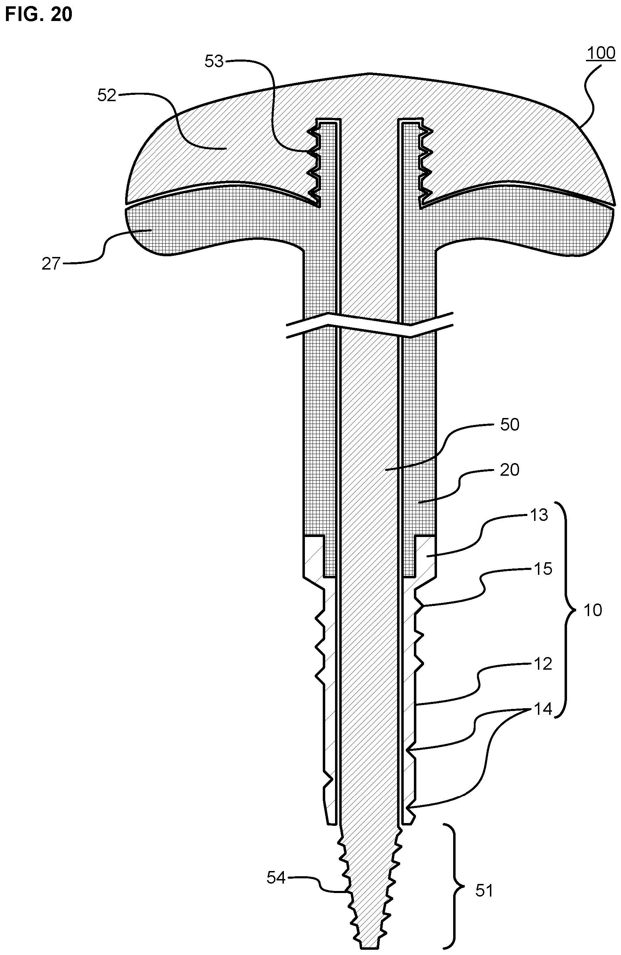

### [3] Threads that tap side wall also on bone cement molding tool (FIG. 20)

In the treatment device of [2], the bone cement molding tool has threads (54) that tap a side wall of the hole and screwed thereinto.

This allows for the formation of a hole with a tapped groove pre-carved in the side wall. When the internal fixation device needs to be removed, the threads for tapping of the bone cement fixation tool are screwed along the aforementioned groove preformed in the hole, allowing the internal fixation device to be screwed in and pulled out with little force and facilitating removal of the bone cement fixation tool after the internal fixation device is removed.

### [4] Screwdriver (FIG. 1, FIG. 2 and FIG. 20)

In [2] or [3], the treatment device (100) further includes screwdriver (20), the internal fixation device being connectable to the screwdriver at the head (13), the screwdriver, when connected to the internal fixation device, communicating with a through hole of the internal fixation device and having a through hole through which tip portions of the bone cement molding tool and the bone cement fixation tool can pass.

This provides a screwdriver (20) that can be used for both the surgery to screw in the internal fixation device (10) and the surgery to remove it.

### [5] Bone cement molding tool can be connected to screwdriver (FIG. 20)

In the treatment device of [4], the bone cement molding tool is inserted into the through hole of the screwdriver connected to the internal fixation device and protrudes a predetermined length from the tip of the internal fixation device.

This allows the bone cement molding tool to be inserted to mold the hole while the screwdriver and internal fixation device are connected together, resulting in the reduced the number of procedural steps.

### [6] Bone cement molding tools and screwdriver are detachable (FIG. 20)

In the treatment device of [5], the bone cement molding tool is detachably connected to the screwdriver.

This allows molding of the hole and the insertion of the internal fixation device into the bone cement can be performed selectively: simultaneously or separately.

### [7] Bone cement molding tool and screwdriver are integrated with screw (FIG. 20)

In the treatment device of [6], the screwdriver has screw structure (53) at the distal end that can be connected to the bone cement molding tool.

This improves operability of the treatment device when molding of the hole and inserting of the internal fixation device into the bone cement are performed at the same time.

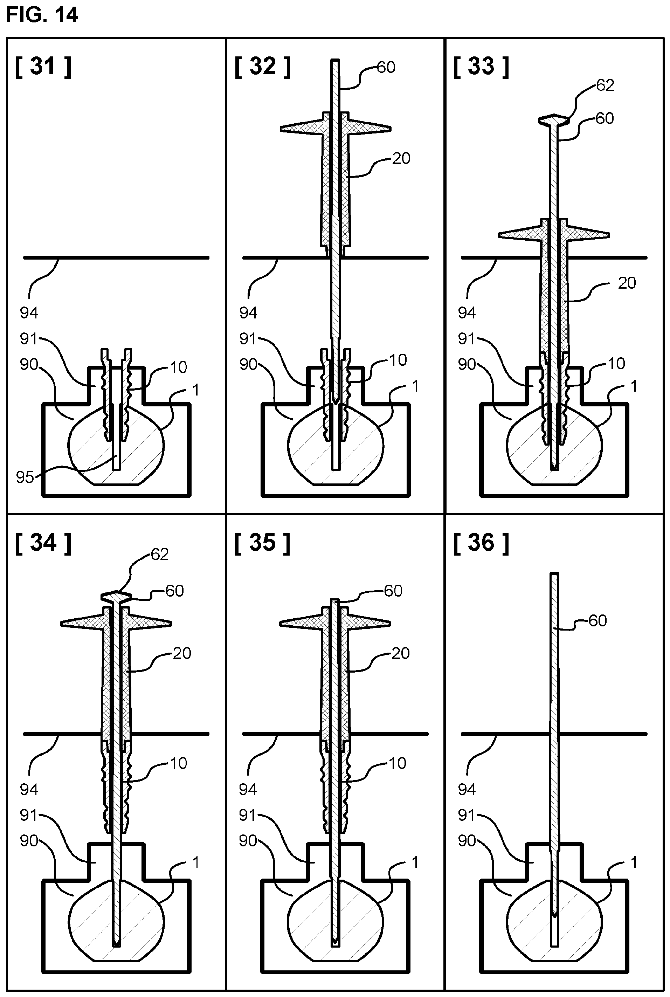

### [8] Bone cement fixation tool with detachable handle (FIG. 14)

In the treatment device of any one of [2] to [7], the bone cement fixation tool has a structure in which handle portion (62) can be detached from a shaft portion.

This allows the handle to be attached when the rotation of the bone cement fixation tool is inhibited and removed when the bone cement fixation tool is pulled out, resulting in increased flexibility in procedure.

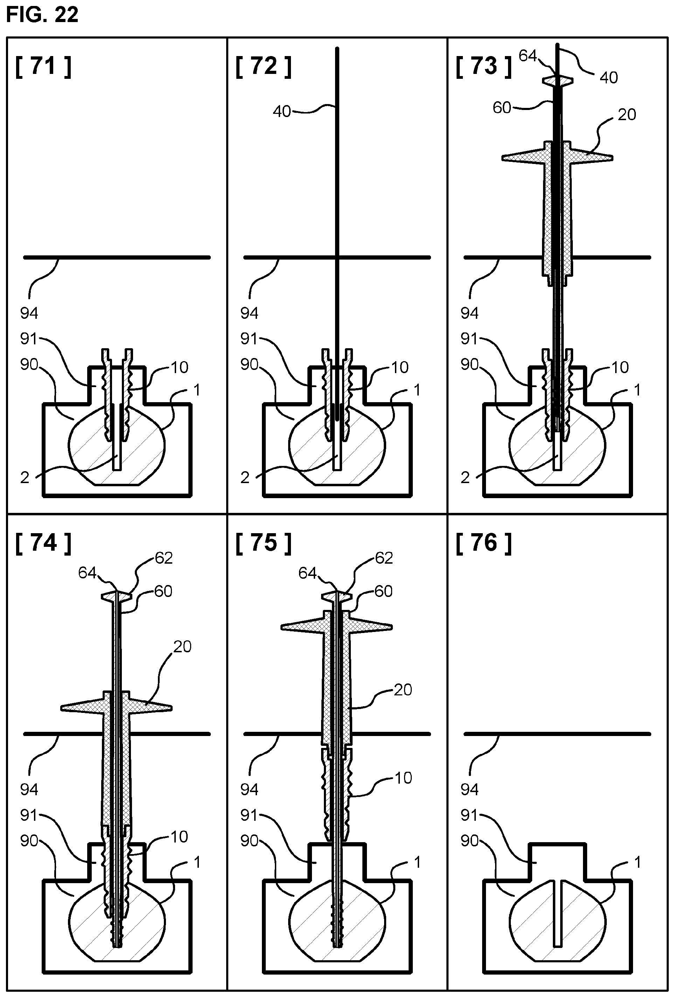

### [9] Guide pin (FIG. 22)

In any one of [2] to [8], the treatment device further includes guide pin (40), and the bone cement fixation tool has through hole (64) through which the guide pin can pass.

This allows bone cement fixation tool to be guided into hole of the affected bone cement along guide pin that has been inserted into the affected area beforehand.

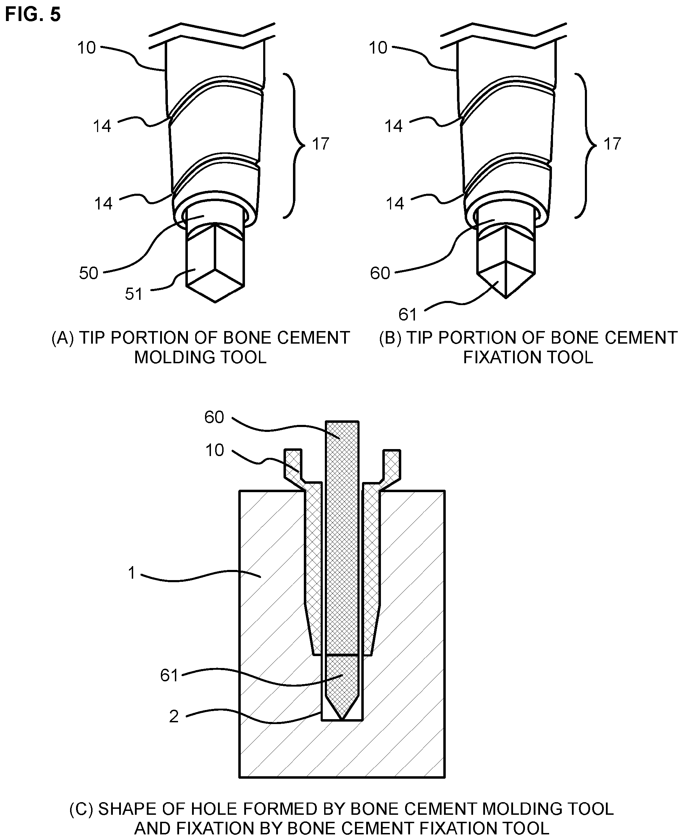

### [10] Bone cement fixation tool (FIG. 5) that contacts side wall of square hole and inhibit rotation of bone cement

In the treatment device of [1], a tip section of the bone cement molding tool is a prism (FIG. 5(a)) or prism tapered (FIG. 5(b)) of which cross-section decreasing toward the tip, and hole (2) which is prism shaped or prism tapered with a decreasing cross-section in the depth direction is molded. the bone cement fixation tool has a tip shape that is able to contact a side wall of the hole and inhibit rotation of the bone cement.

This allows the bone cement fixation tool to contact the side wall of the hole and reduce rotation of the bone cement. The bone cement fixation tool is simply inserted into the hole and can be easily pulled out.

### [11] Bone cement fixation tool with detachable handle (FIG. 14)

In the treatment device of [10], the bone cement fixation tool has a structure in which handle portion (62) can be detached from a shaft portion.

This allows the handle to be attached when the rotation of the bone cement fixation tool is inhibited and removed when the bone cement fixation tool is pulled out, resulting in increased flexibility in procedure.

### [12] Screwdriver

In [10] or [11], the treatment device further includes a screwdriver, the internal fixation device being connectable to the screwdriver at the head, the screwdriver, when connected to the internal fixation device, communicating with the through hole of the internal fixation device and having a through hole through which tip portions of the bone cement molding tool and the bone cement fixation tool can pass.

This provides a screwdriver (20) that can be used for both the surgery to screw in the internal fixation device (10) and the surgery to remove it.

### [13] Guide pin (FIG. 22)

In any one of [10] to [12], the treatment device further includes guide pin (40), and the bone cement fixation tool has through hole (64) through which the guide pin can be caused to pass.

This allows bone cement fixation tool to be guided into hole of the affected bone cement along guide pin that has been inserted into the affected area beforehand.

### 2. Details of the embodiments

Details of the embodiments will be described in more detail.

### [Embodiment 1]

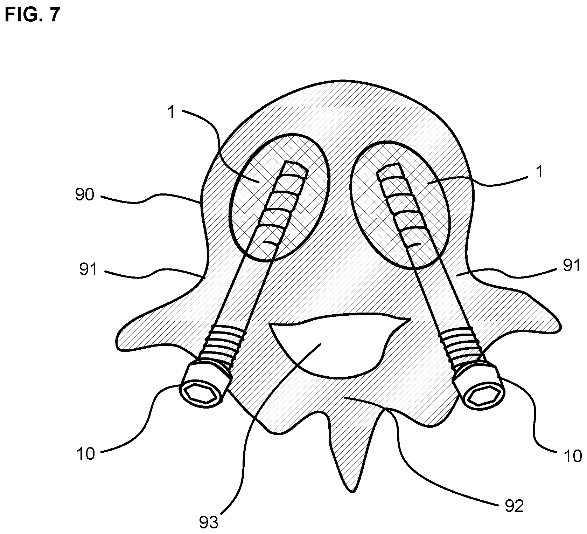

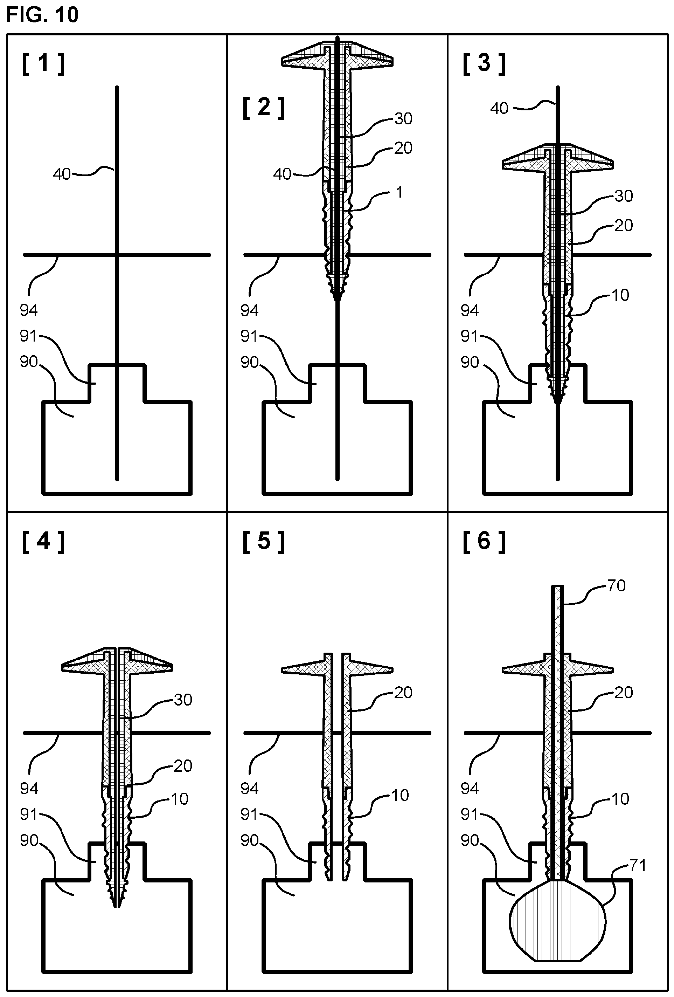

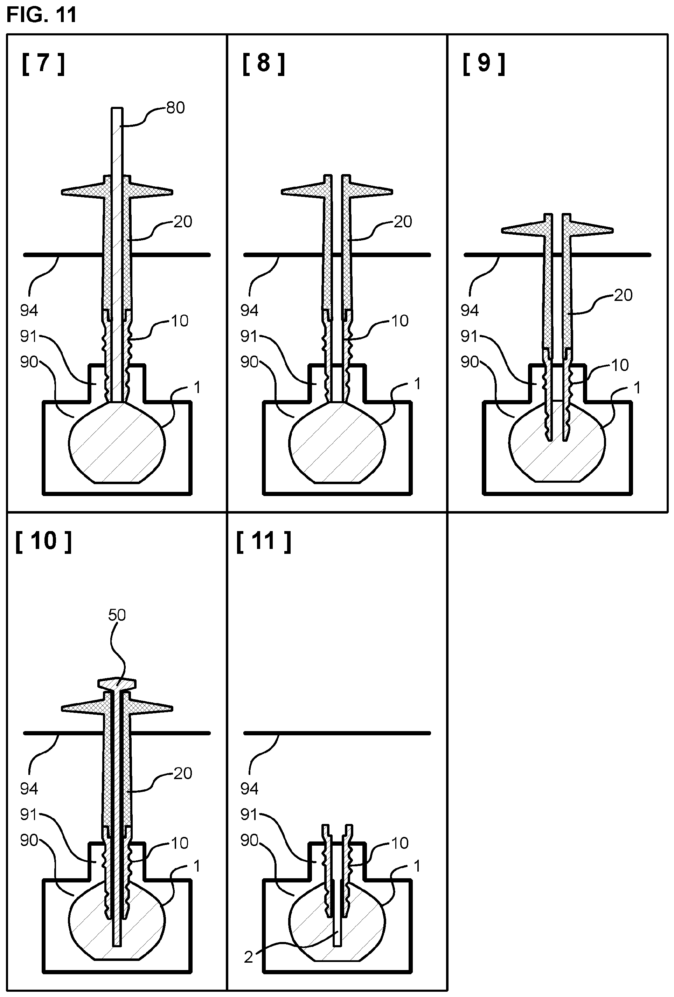

FIG. 7 is an explanatory drawing overviewing an example use of internal fixation device 10. The spine, in the patient's standing position, consists of a plurality of vertically stacked vertebral bodies 90, one of vertebral bodies 90 is shown in FIG. 7 as if seen in perspective from above and below. Vertebral body 90 is supported by a pair of pedicles 91 on each side, which are connected by vertebral arch 92. The area bounded by vertebral body 90, pedicles 91, and vertebral arch 92 is spinal canal 93. A compression fracture is a condition in which the vertebral body is compressed and crushed by forces in the vertical direction. In the treatment using internal fixation device 10 according to the present invention, as described below with reference to FIGS. 10 and 11, a space is re-formed in vertebral body 90 and bone cement 1 is injected thereinto, and before it hardens, internal fixation device 10 is inserted (screwed) and supported from pedicle 91 side. In other words, internal fixation device 10 is inserted through pedicle 91 into bone cement 1 pre-filled into vertebral body 90, and as bone cement 1 hardens, it is supported by internal fixation device 10 from pedicle 91 and fixed into vertebral body 90. Internal fixation device 10 according to the present invention is an instrument that is screwed into bone cement 1 in vertebral body 90 while tapping the bone tunnel formed in pedicle 91. Internal fixation device 10 may be a conventional screw as described in PTL 1, for example, but the threads for tapping on the tip side that is embedded in bone cement 1 is not necessary.

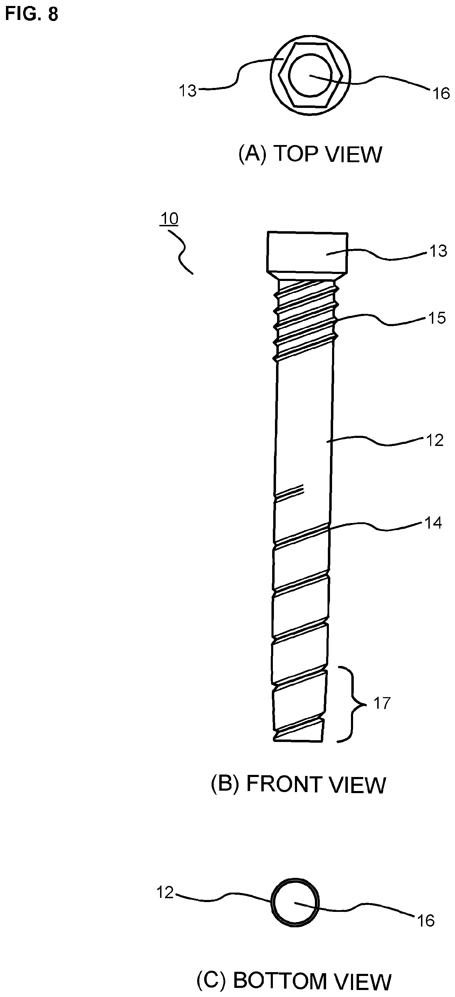

FIG. 8 shows a top view (a), a front view (b), and a bottom view (c) of an example configuration of an easy-to-pull out internal fixation device 10, which has been filed internationally as PCT/JP2023/22593. Since the back view and left/right side views are the same as the front view (b), except that grooves 14 and threads 15 are placed at extended positions from the front view (b), illustration thereof are therefore omitted. Internal fixation device 10 has a round column shaft 12 and connection 13 that is connectable to a screwdriver at a distal end of shaft 12. Shaft 12 has helical grooves 14 on a proximal side. Suitably shaft 12 is cylindrical in shape as illustrated in the figures, and has through hole 16 that passes from the distal end to the proximal end along a central axis. Through hole 16 can, for example, allow a guide pin to pass through. Through hole 16 may be configured to allow passage of a bone drilling tool, a balloon catheter, a bone cement injector, and/or the like. Connection 13 of the head is configured as a hexagonal prismatic recess to engage with a screwdriver (not shown) and to engage with for example a hexagonal wrench so as to rotate the entire internal fixation device 10. Although FIG. 8 shows an example of a hexagonal prism-shaped head (connecting section), assuming connection with a hexagonal wrench-shaped screwdriver head, the shape of the head can be arbitrarily determined as long as it can transmit rotational force, and may be changed to star-shape, triangle, square, or cross-shape, for example. The reference sign 13 may be used herein to refer to as the "head" with emphasis on its position in internal fixation device 10, or as the "connection" with emphasis on its function of connecting to the screwdriver.

While the depth of grooves 14 of internal fixation device 10 can be uniform, it is more suitable if it is deeper on the proximal (tip) side than on the distal (head) side of shaft 12. Since the proximal side (tip side) that initially contacts bone cement 1 is deeper, when internal fixation device 10 is screwed into bone cement 1 while rotating along the spiral of grooves 14, bone cement 1 enters deeper into grooves 14, and as it is screwed, is fed along the spiral of grooves 14 to the distal side (head side) where grooves 14 are shallower. So, bone cement 1 smoothly enters grooves 14 and is securely fixed. During removal, internal fixation device 10 is separated from bone cement 1 while rotating reversely along the spiral of grooves 14, so the force required for removal can be reduced.

More suitably, shaft 12 of internal fixation device 10 has tapered section 17 that gradually decreases in diameter from the center toward its proximal end. When internal fixation device 10 is screwed into bone cement 1, the resistance when first inserting it into bone cement 1 is reduced, and the force required to remove it is also reduced.

The distal side (head side) of shaft 12 of internal fixation device 10 should be provided with threads 15. Threads 15 is a spiral protrusion protruding outward from the outer surface of shaft 12 and functions to cause internal fixation device 10 (shaft 12) to advance along its central axis by tapping the surrounding bone as it rotates. Threads 15 advances by tapping pedicle 91, securely fixing internal fixation device 10 and stabilizing bone cement 1 in vertebral body 90 into which shaft 12 has been inserted.

Threads 15 and grooves 14 are suitably formed to have the same lead. Lead is the travel distance of shaft 12 along the central axis relative to the amount of rotation, and it is desirable that the travel distance that shaft 12 advances while threads 15 taps pedicle 91 and the travel distance that shaft 12 advances along the helix of grooves 14 when it is screwed into bone cement 1 should match. However, the travel distances do not necessarily need to match precisely, and can be intentionally made different. For example, by setting the lead of grooves 14 larger than the lead of threads 15, grooves 14 at the tip of shaft 12 is partially screwed into bone cement 1, and after threads 15 begins tapping the pedicle, the force can be exerted to bring bone cement 1 toward the pedicle as internal fixation device 10 (shaft 12) is rotated.

The number grooves 14 can be different from the number of ridges in threads 15. For example, the number of grooves 14 should be smaller than the number of ridges in threads 15. This allows for proper design of the bonding force between internal fixation device 10 and bone cement 1 while maintaining a secure fixation by threads 15 with pedicle 91. As described above, it is suitable that grooves 14 and threads 15 are formed to have the same lead, which creates a new challenge of setting the appropriate contact area with the object, but that challenge is solved by designing the number of grooves and the number of the threads independently. Reducing the number of grooves 14 reduces the area of contact with bone cement 1, thereby reducing the force required for pulling out. On the other hand, by increasing the contact area between pedicle 91 and threads 15, the fixation of internal fixation device 10 to pedicle 91 can be strengthened. By optimizing the number of grooves and/or the threads rather than the pitch, the relationship between bone cement 1 and grooves 14 and the relationship between pedicle 91 and threads 15 can be optimized independently.

The term "internal fixation device" herein should include a device that is inserted from the pedicle into the bone cement pre-filled into the vertebral body and is fixed from the pedicle into the bone cement vertebral body when the bone cement hardens. Thus, the internal fixation device herein includes not only the easy-to-pull out internal fixation device 10 filed internationally as PCT/JP2023/022593, but also those devoid of grooves 14, of threads 15, of both grooves 14 and threads 15, or even conventional screws threaded on the entire surface.

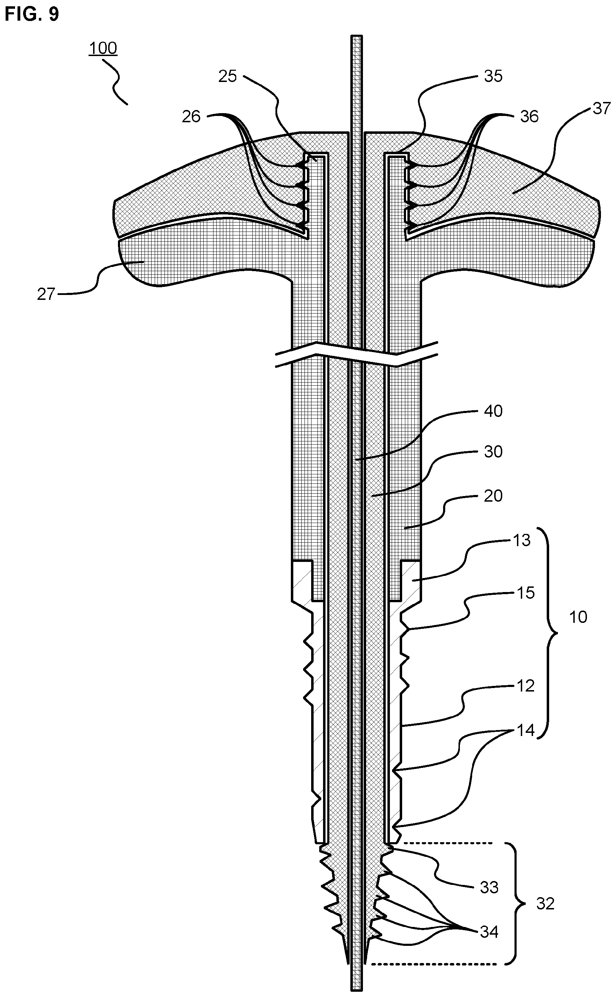

FIG. 9 is an explanatory diagram schematically illustrating a cross-sectional structure to show an example configuration of treatment device 100. In FIG. 9, the scale in the direction of the central axis (vertical direction on the paper) is compressed, and the direction along the patient's body surface (horizontal direction on the paper) is emphasized. Treatment device 100 includes a cylindrical screwdriver 20 that can screw internal fixation device 10 into a bone, and an inner cylinder 30 that is inserted into screwdriver 20, and is configured as follows.

Internal fixation device 10 has a rounded tip to allow insertion and removal of flexible vertebral body drilling tools such as balloons, and has threads 15 that can tap bone or cement on an outer wall, and through hole 16 that allows passage of inner cylinder 30 from head 13 to tip along the central axis. Screwdriver 20 is configured to fit into head 13 of internal fixation device 10 and connect to internal fixation device 10 by moving it along the central axis, thereby transmitting to internal fixation device 10 a force in the direction of rotation around the central axis. For example, the tip of screwdriver 20 should have a hexagonal wrench shape (hexagonal prism) and head 13 of internal fixation device 10 may desirably have a hexagonal groove (recess). As mentioned above citing FIG. 8, the shape of the screwdriver tip and the shape of the screw head can be those other than hexagonal, such as star-shape, as long as engagement is achieved upon insertion. Sliding screwdriver 20 along the central axis into the head of internal fixation device 10 causes the tip of screwdriver 20 to fit into head 13 of internal fixation device 10, allowing the screw to be screwed in by screwdriver 20, resulting in easy removal by simply pulling out screwdriver 20.

Inner cylinder 30 has tip section 32 that is inserted from the distal end of screwdriver 20 through the through holes of screwdriver 20 and internal fixation device 10 and protrudes proximally than the tip of internal fixation device 10. Inner cylinder 30 is connected to and integrated with internal fixation device 10 and screwdriver 20 with tip section 32 protruding proximally from the tip of internal fixation device 10, resulting in improved operability in, for example, screwing internal fixation device 10. Also, the insertion of guide pin 40 strengthens the integration and makes it impossible to pull inner cylinder 30 out of internal fixation device 10 and screwdriver 20.

An example structure for integrating internal fixation device 10, screwdriver 20, and inner cylinder 30 will be described.

The tip section 32 of inner cylinder 30 is divided into a plurality of tongue pieces 31 in the direction along the central axis, and each of the plurality of tongue pieces 31 has protrusion 33 in the direction away from the central axis. Protrusion 33 has the function of a notch that regulates internal fixation device 10 from being pulled out of inner cylinder 30, as described below, so the reference sign 33 is referred to as a notch. The plurality of tongue pieces 31 are formed, for example, by dividing cylindrical inner cylinder 30 with slits from the tip side. The number of divisions can be arbitrarily set to, for example, 4, 6, 2, or 3. In addition, some processing (e.g., heat treatment, reduction in thickness, laminating highly elastic metal materials) may be applied to provide adequate elasticity. Elasticity of tongue piece 31 is designed to be moderate. For example, elasticity is designed such that when inner cylinder 30 is passing through the through hole in screwdriver 20, notch 33 is pressed against the inner wall of the through hole to flex in the central axial direction to allow the passage of inner cylinder 30, and when notch 33 exits proximal to the tip of internal fixation device 10, the flexure is restored and notch 33 is caught on the tip of internal fixation device 10. When notch 33 is smooth in shape, it can be configured so that even after notch 33 exits the tip of internal fixation device 10, the force to pull inner cylinder 30 can still pull notch 33 into through hole 16 of internal fixation device 10 and inner cylinder 30 can be pulled out by flexing tongue piece 31 in the central axis direction again. The insertion of guide pin 40 makes it impossible to bend tongue piece 31 in the central axial direction, making it impossible to pull inner cylinder 30 out of internal fixation device 10 and screwdriver 20.

Furthermore, as shown in FIG. 9, it is more suitable if screwdriver 20 and inner cylinder 30 are configured so that they can be connected at their distal ends. The distal end of screwdriver 20 is provided with a cylindrical connection 25 with external threads 26 on its outer circumference, and the distal end of inner cylinder 30 has a groove-shaped connection 35 that can accommodate the cylinder and is provided with internal threads 36 that engage with the external threads 26. The relationship between recess and protrusion, and external threads and internal threads may be reversed, or other connection mechanisms may be used. As illustrated in FIG. 9, screwdriver 20 has handle 27, and inner cylinder 30 has handle 37, these handles being preferably integrated for easy gripping when connected. In addition to threading mechanism as in this example, a latch mechanism (not shown) may be provided to prevent removal of the connection.

Treatment device 100 according to the present invention includes a bone cement molding tool and a bone cement fixation tool, which are inserted into an internal fixation device that supports and internally fixes bone cement filled into a vertebral body from a pedicle and is configured as follows.

The first premise is that internal fixation device 10 has a through hole 16 in the direction of the central axis from head 13 to the tip (proximal end) so that it can be inserted and removed from bone cement 1 along the central axis. As an example, see the explanation above, citing FIG. 8.

FIG. 1 is an explanatory drawing schematically illustrating a cross-sectional structure to show an example configuration of a treatment device 100 according to the present invention, including a bone cement molding tool 50. In FIG. 1, the scale in the direction of the central axis (vertical direction on the paper) is compressed, and the direction along the patient's body surface (horizontal direction on the paper) is emphasized. Internal fixation device 10 and screwdriver 20 each have a through hole along the central axis, and when connected, the through holes are connected to each other. Bone cement molding tool 50 is configured to be inserted from the distal end into the through hole that is connected when internal fixation device 10 and screwdriver 20 are connected, tip section 51 protruding proximally from the tip of internal fixation device 10. Bone cement molding tool 50 is inserted into bone cement 10 through through hole 16 to form a predetermined shaped hole 2 after internal fixation device 10 is inserted into bone cement 1. While bone cement molding tool 50 shown in FIG. 1 has handle 52, rotating bone cement molding tool 50 is not important, rather, more important is the action of restricting tip section 51 from going too deep; so, handle shape is not necessary. Tip section 51 is designed to protrude from the tip section of internal fixation device 10 to the desired depth when hole 2 is molded in bone cement 1. The desired depth is arbitrary, e.g., about 1 cm. A scale can be engraved or printed on the distal end side of the shaft of bone cement molding tool 50 to indicate the amount of protrusion, and a stopper with a diameter larger than the through hole of screwdriver 20 can be provided to prevent extreme protrusion. Also, the position at which the handle 52 is mounted can be adjusted so that the handle 52 serves as the stopper. Alternatively, a structure in which bone cement molding tool 50 could be connected to screwdriver 20 with a screw or other means allows internal fixation device 10 to be screwed into the bone cement while molding hole 2 at the same time. Further alternative structure is that the depth of hole 2 does not have to be controlled by bone cement molding tool 50 alone, but once the length of internal fixation device 10 is inserted to the assumed depth, hole 2 will naturally molded to a predetermined depth (e.g., 1 cm deeper than the tip of internal fixation device 10).

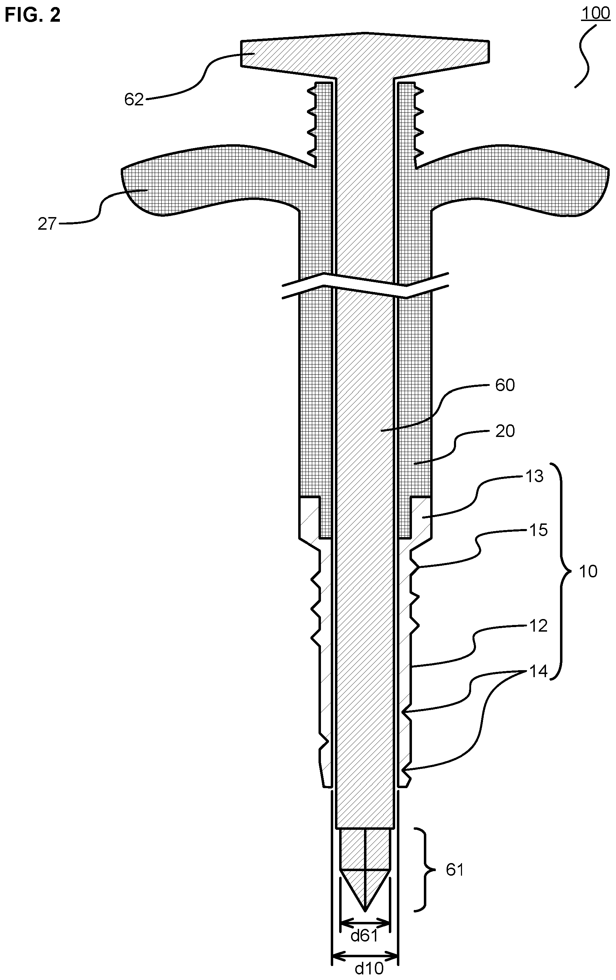

FIG. 2 is an explanatory drawing schematically illustrating a cross-sectional structure to show an example configuration of a treatment device according to the present invention, including bone cement fixation tool 60. As in FIG. 1, the scale in the direction of the central axis (vertical direction on the paper) is compressed, and the direction along the patient's body surface (horizontal direction on the paper) is emphasized. Similar to bone cement molding tool 50, bone cement fixation tool 60 is configured to be inserted from the distal end into the through hole that is connected when internal fixation device 10 and screwdriver 20 are connected, tip section 61 protruding proximally from the tip of internal fixation device 10. Both the through hole in communication and bone cement fixation tool 60 to be inserted are circular in cross section, so that their mutual rotational movements do not interfere with each other. In simple terms, rotating screwdriver 20 does not rotate bone cement fixation tool 60. Bone cement fixation tool 60 has a tip section 61 that is inserted into hole 2 formed by bone cement molding tool 50. Tip section 61, for example is in the same shape as the aforementioned predetermined shape; tip section 61 is inserted into bone cement 1 with holes 2 through the through holes 16 of internal fixation device 10, and is inserted into the holes 2, thereby inhibiting rotation around the central axis of bone cement 1. The "same shape" is not intended to mean that the size is the same without error, but it is enough that tip section 61 of bone cement fixation tool 60 should fit into hole 2 molded by tip section 51 of bone cement molding tool 50, inhibiting the rotation of bone cement 1; rather, it is desirable to allow for a tolerance for smooth insertion. This is true throughout this document. Since tip section 61 of bone cement fixation tool 60 should fit into hole 2 molded by tip section 51 of bone cement molding tool 50, the "same shape" is a sufficient but not a necessary condition, and various shapes can be employed as described below. When tip section 61 of bone cement fixation tool 60 fits into hole 2 molded by tip section 51 of bone cement molding tool 50, the tip thereof contacts the bottom of hole 2, inhibiting movement in the direction of pulling out bone cement 1.

This provides a treatment device that assists in the removal of an internal fixation device that is inserted through a pedicle into bone cement 10 pre-filled into the vertebral body.

Bone cement fixation tool 60 is more suitably provided with a handle 62 at the distal end thereof. As mentioned above, screwdriver 20 and the inserted bone cement fixation tool 60 are configured so that their rotational movements do not interfere with each other. This allows that when screwdriver 20 is rotated, bone cement fixation tool 60 can be held back by handle 62 to prevent them from rotating together. Handle 62 may be configured to be detachable, as described below.

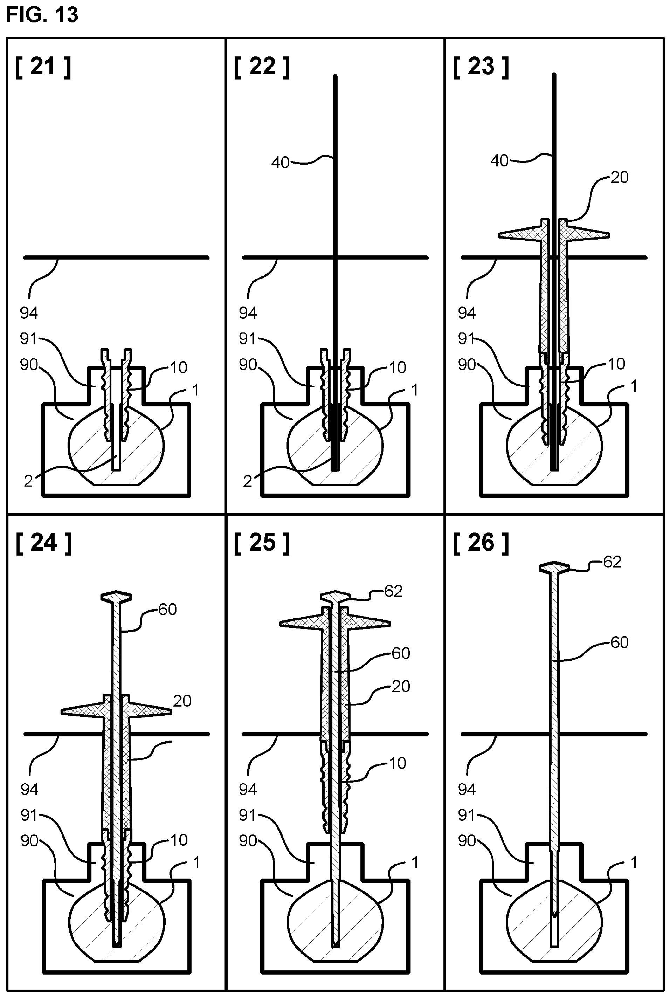

Although FIG. 2 depicts tip section 61 of bone cement fixation tool 60 protruding slightly from the tip of internal fixation device 10 and handle 62 touching the distal end of screwdriver 20, the actual length of bone cement fixation tool 60 is longer than the illustrated example. As discussed below, citing FIG. 13, with tip section 61 of bone cement fixation tool 60 inserted into hole 2 of bone cement 1, handle 62 is positioned distally away for removal of internal fixation device 10 (see FIG. 13[24] and [25]). A scale may be provided on the distal side of bone cement fixation tool 60 to indicate how much tip section 61 of bone cement fixation tool 60 protrudes beyond the tip section of internal fixation device 10 when screwdriver 20 and internal fixation device 10 are connected. When bone cement fixation tool 60 is inserted into internal fixation device 10 before removal, the insertion of bone cement fixation tool 60 stops in hole 2 formed in bone cement 1; however, in the unlikely event that bone cement 1 is damaged and does not stop in hole 2, attention can be called so that no damage to surrounding body tissues due to insertion of a longer length than expected. A stopper mechanism may also be provided to prevent internal fixation device 10 too deep.

The shapes of tip section 51 of bone cement molding tool 50 and tip section 61 of bone cement fixation tool 60 will now be described in more detail. The aforementioned "predetermined shape" is suitably a prism-shape, for example.

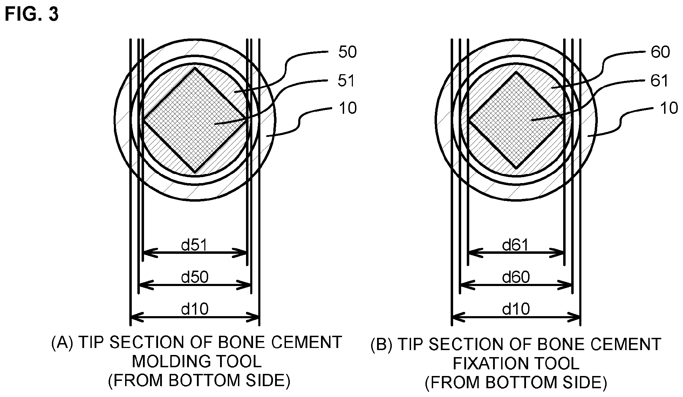

FIGS. 3 and 5 each is an explanatory drawing illustrating the tip portions of bone cement molding tool 50 and bone cement fixation tool 60 to show examples of their configurations((a) shows tip section 51 of bone cement molding tool 50 and (b) shows tip section 61 of bone cement fixation tool 60). FIG. 3 shows the bottoms viewed from the proximal end side and FIG. 5 shows overviewed appearance and cross-sectional structure of each tip portion. As an example, bone cement molding tool 50 has a round column shaft and a quadrangular prism-shaped tip section 51, while bone cement fixation tool 60 has a round column shaft and a quadrangular prism -shaped tip section 61. As shown in the figure, tip section 61 of bone cement fixation tool 60 is more suitable if there is an additional square pyramid at the end of the quadrangular prism, which allows for smooth insertion. The diameter d50 of the round column shaft of bone cement molding tool 50 is slightly smaller than the diameter d10 of through hole 16 of bone cement 10, and the quadrangular prism of tip section 51 is formed inside the round column shaft. For example, the diagonal length d51 of the square of the cross section of a regular quadrangular prism is equal to or smaller than d50. Bone cement fixation tool 60 has a round column shaft and a quadrangular prism-shaped tip section 61. The diameter d60 of the round column shaft of bone cement fixation tool 60 is slightly smaller than the diameter d10 of through hole 16 of bone cement 10, and the quadrangular prism of tip section 61 is formed inside the round column shaft. For example, the diagonal length d61 of the square of the cross section of a regular quadrangular prism is equal to or smaller than d51, and should be large enough to fit approximately tightly into hole 2 formed by tip section 51 of bone cement molding tool 50. Since it is sufficient to fit and prevent bone cement 1 from rotating, there may be some tolerance rather than a snug fit.

This allows bone cement fixation tool 60 to inhibit rotation of the bone cement by simply inserting it into the hole formed in bone cement 1.

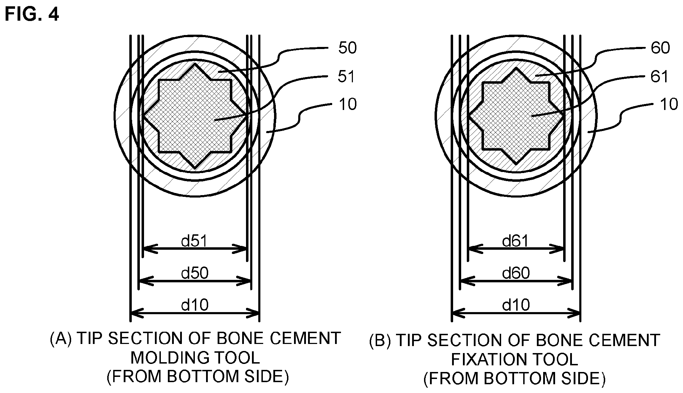

Although quadrangular prism is used as an example, other shapes may be used. It can be triangular prism, pentagonal prism, or hexagonal prism, and can also be star-shaped, plate-shaped, cross-shaped, or any other shape. It is sufficient if bone cement molding tool 50 and bone cement fixation tool 60 have the same shaped tip section and tip section 61 of bone cement fixation tool 60 fits into hole 2 formed by bone cement molding tool 50 to inhibit movement in the rotational direction. FIG. 4 is an explanatory drawing illustrating the bottoms of tip sections (star-shape) viewed from the proximal end to show another example configuration of bone cement molding tool 50 and the bone cement fixation tool 60.

FIG. 5 also shows an example where bone cement molding tool 50 and bone cement fixation tool 60 have the same quadrangular prism-shaped tip section and fit tightly together to inhibit movement in the rotational direction. As shown in the cross-sectional structure in FIG. 5(c), it is understood that the movement of bone cement 1 in the direction of pulling bone cement 1 out is also inhibited by the tip of bone cement fixation tool 60 abutting the bottom of hole 2, but inhibition of rotation is main purpose here.

FIG. 6 is an explanatory drawing illustrating the overviewed bottoms and cross-sectional structure of the tip sections of bone cement molding tool 50 and the bone cement fixation tool 60 to show another example configuration thereof. Tip section 51 of bone cement molding tool 50 and tip section 61 of bone cement fixation tool 60 are prism tapered with a square pyramid formed at the end of the quadrangular prism. Tip section 51 of bone cement molding tool 50 is more acute than tip section 61 of bone cement fixation tool 60, and as shown in the cross-sectional structure in FIG. 6(c), before reaching the bottom of hole 2 formed by tip section 51 of bone cement molding tool 50, tip section 61 of bone cement fixation tool 60 abuts against the side wall and fit into there. This inhibits movement in both the rotational and pull-out directions.

While the cone tips are drawn sharp to aid understanding in FIGS. 5 and 6, it is desirable that the tips should be rounded so as not injure any part of the body tissue, nerves or blood vessels.

Furthermore, tip section 61 of bone cement fixation tool 60 does not need to have the same shape as tip section 51 of bone cement molding tool 50, as described below in Embodiments 2 and 3, and it is sufficient that tip section 61 can fit into hole 2 formed in bone cement 1 by bone cement molding tool 50 and inhibit rotation of bone cement 1.

A surgical procedure using treatment device 100 according to the present invention will now be described.

FIGS. 10 and 11 are explanatory drawings schematically illustrating the procedure of implanting internal fixation device 10 in the treatment using treatment device 100, with FIG. 10 and FIG. 11 showing the first half and the second half of the procedure, respectively. Although FIGS. 10 and 11 illustrate vertebroplasty, the same method of inserting internal fixation device 10 into the vertebral body is also used for vertebral augmentation in spinal fusion and artificial intervertebral disc replacement (not shown). FIGS. 10 and 11 schematically show a cross-section of the patient's affected area from the outer skin 94 through pedicle 91 to vertebral body 90, with the upper side of the paper as distal side and the lower side as proximal side. The scale in the direction of the central axis (vertical direction on the paper) is compressed, and the direction along the patient's body surface (horizontal direction on the paper) is emphasized. Since pedicles 91 are on each side of the vertebral bodies 90, the same procedure is performed from the left and right for one vertebral body 90, FIGS. 10 and 11 show one of them.

Step 1 ([1]): Incise and drill subcutaneous tissue from the patient's back body surface, i.e., skin 94, to the affected vertebral body 90, and introduce guide pin 40 through pedicle 91 to vertebral body 90.

Step 2 ([2]): Connect internal fixation device 10 and screwdriver 20, insert inner cylinder 30 into an inner through hole of internal fixation device 10, combine inner cylinder 30 with the tip section 32 protruding from the tip of internal fixation device 10, and insert inner cylinder 30 into the patient's body along guide pin 40, covering guide pin 40.

Step 3 ([3]): Push treatment device 100, in which internal fixation device 10, screwdriver 20, and inner cylinder 30 are connected and integrated, further proximally along guide pin 40, and screw tips of inner cylinder 30 and internal fixation device 10 into pedicle 91.

Step 4 ([4]): After screwing the tip of internal fixation device 10 until it reaches near the entrance of vertebral body 90, remove guide pin 40. At this time, notch 33 of inner cylinder 30 is in contact with the tip of internal fixation device 10 to deter inner cylinder 30 from spontaneously pulling out, but guide pin 40 has been pulled out, and the force to pull out inner cylinder 30 allows tongue piece 31 to flex in the central axis direction to pull out inner cylinder 30.

Step 5 ([5]): Remove inner cylinder 30 At this time, screwdriver 20 is simply inserted into head 13 of internal fixation device 10, and is held down so that it cannot be pulled out. Although not specifically illustrated, this step may introduce vertebral body drilling tool to drill within vertebral body 90 to fill cement for reinforcement through the through holes of screwdriver 20 and internal fixation device 10, if necessary.

Step 6 ([6]): introduce balloon catheter 70 into vertebral body 90 through the through holes of screwdriver 20 and internal fixation device 10, and inflate balloon 71 in vertebral body 90. For example, balloon 71 is inflated by injecting a contrast medium through balloon catheter 70 and applying pressure. The purpose is to restore vertebral bodies 90 to their original size when vertebral bodies 90 are compression fractured due to osteoporosis or other causes.

Step 7 ([7]): Pull balloon catheter 70 out of the through holes of screwdriver 20 and internal fixation device 10 and insert cement injector 80 to inject bone cement 1 into vertebral body 90. Since the volume of cement that can be filled at one time by cement injector 80 is about 1.5 ml, for example, in the case of vertebroplasty for a fracture, for example, about 10 ml of bone cement 1 is filled into vertebral body 90 by repeatedly delivering cement 6 to 7 times in total, about 3 times from each side. When applied to vertebral body augmentation procedures other than vertebroplasty, spinal fusion, and artificial intervertebral disc replacement, the amount of cement to fill should be adjusted according to the bone condition.

Step 8 ([8]): Pull cement injector 80 out through the through holes of screwdriver 20 and internal fixation device 10.

Step 9 ([9]): Screw internal fixation device 10 into the filled bone cement 1 using screwdriver 20.

Step 10 ([10]): Insert bone cement molding tool 50 into bone cement 1 through the through holes of screwdriver 20 and internal fixation device 10. Bone cement 1 has not yet hardened, and hole 2 of a predetermined shape is molded by tip section 51 of bone cement molding tool 50.

Step 11 ([11]): Pull bone cement molding tool 50 and screwdriver 20 out and suture incised skin 94 to complete the treatment.

As described above, an important part of the present invention is, in the procedure for internal fixation of bone cement 1 with the implantation internal fixation device 10, to mold hole 2 of a predetermined shape in bone cement 1 that has not yet hardened by tip section 51 of bone cement molding tool 50. The procedure for implanting the internal fixation device described here, citing FIGS. 10 and 11, is only one example. Steps 2 ([2]) through 6 ([6]) may employ a procedure where, for example, a balloon is inserted over another outer cylinder to secure the space to be filled with bone cement 1, a thick guide pin is inserted, the outer cylinder is removed, and internal fixation device 10 and screwdriver 20 are inserted.

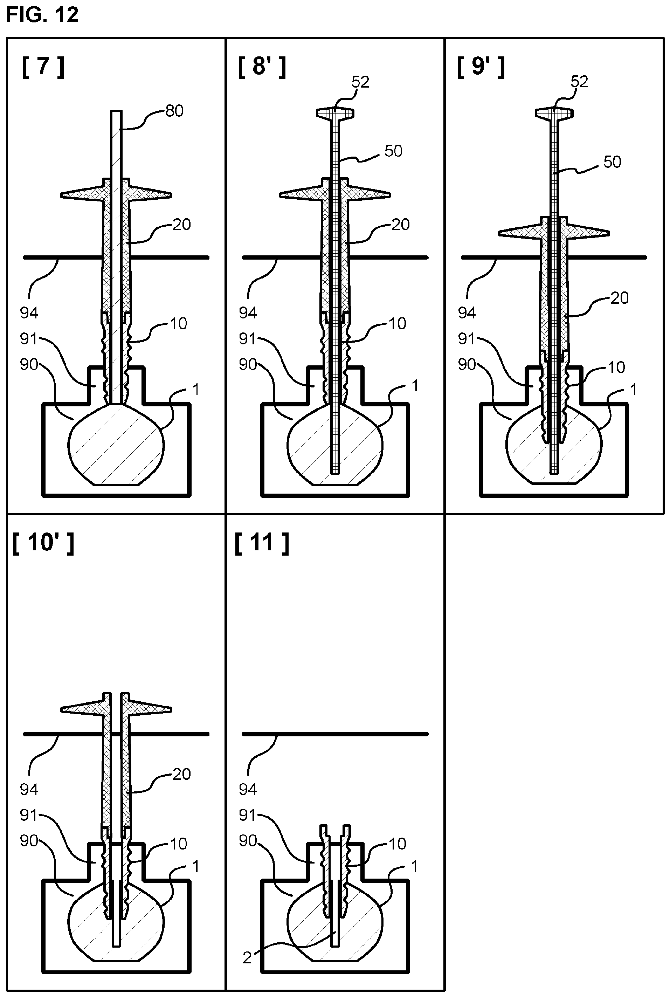

FIG. 12 is an explanatory drawing schematically illustrating another example of the second half of the treatment procedure of implanting the internal fixation device in treatment using the treatment device according to the present invention. The first half (Steps 1 through 6) through Step 7 are the same as described above, citing FIGS. 10 and 11.

Step 8 ([8']): Pull cement injector 80 through the through holes of screwdriver 20 and internal fixation device 10 and insert bone cement molding tool 50. Tip section 51 of bone cement molding tool 50 molds a predetermined shaped hole 2 in bone cement 1.

Step 9 ([9']): Screw internal fixation device 10 into the filled bone cement 1 using screwdriver 20.

Step 10 ([10']): Pull bone cement molding tool 50 out. Bone cement 1 has hole 2 of a predetermined shape molded in it.

Step 11 ([11]): Pull screwdriver 20 out (in practice, along with pulling out of bone cement molding tool 50 in step 10 ([10'])) and suture incised skin 94 to complete the treatment.

Thus, the molding of the hole 2 of predetermined shape in bone cement 1 by bone cement molding tool 50 can be performed either before or after the insertion of internal fixation device 10 into bone cement 1 using screwdriver 20. According to the procedure in FIG. 11, after internal fixation device 1 is inserted into bone cement 1, hole 2 is molded by bone cement molding tool 50, so the misalignment of through hole 16 of internal fixation device 1 and hole 2 can be minimized. In the procedure in FIG. 12, hole 2 is molded by bone cement molding tool 50 before bone cement 1 hardens, and then internal fixation device 1 can be inserted subsequently.

The procedure for removing internal fixation device 10 will now be described.

FIG. 13 is an explanatory drawing schematically illustrating an example of a procedure of removing the internal fixation device 10 in treatment using the treatment device according to the present invention.

Initial state ([21]): In the patient's vertebral body 90, bone cement 1 is internally fixed by being supported by internal fixation device 10 inserted through pedicle 91. Hole 2 in predetermined shape is formed in bone cement 1 through through hole 16 in internal fixation device 10.

Step 1 ([22]): Incise and drill subcutaneous tissue from the patient's back body surface, i.e., skin 94, to the affected vertebral body 90, and insert guide pin 40 in through hole 16 of internal fixation device 10 inserted in pedicle 91. Although FIG. 13[22] illustrates a state in which guide pin 40 is introduced to reach hole 2 formed in bone cement 1 in vertebral body 90, guide pin 40 only needs to reach through hole 16 of internal fixation device 10.

Step 2 ([23]): Guide screwdriver 20 to internal fixation device 10 by aligning the through hole of screwdriver 20 with the inserted guide pin 40, and connect the tip of screwdriver 20 by fitting it into head (connection) 13 of internal fixation device 10.

Step 3 ([24]): Insert bone cement fixation tool 60 into the through hole of screwdriver 20 and through hole 16 of internal fixation device 10, which are in communication when screwdriver 20 and internal fixation device 10 are connected, and guide bone cement into vertebral body 90, inserting tip section 61 into hole 2 of bone cement 1. The shape of tip section 61 of bone cement fixation tool 60 and the shape of hole 2 of bone cement 1 are the same so that they fit together, resulting in inhibited movement in the rotational direction. The movement in the direction of pulling out bone cement 1 is inhibited by the tip of bone cement fixation tool 60 contacting the bottom of hole 2.

Step 4 ([25]): with tip section 61 of bone cement fixation tool 60 fit into hole 2 of bone cement 1 and inhibited rotation and movement in the direction of pulling out, rotate screwdriver 20 to remove internal fixation device 10 while holding bone cement fixation tool 60 with handle 62 to prevent it from rotating. If the threads 15 on the distal side of internal fixation device 10 tapped pedicle 91 during insertion, then by rotating the device in the reverse direction of insertion, internal fixation device 10 can be given driving force in the removal direction, allowing internal fixation device 10 to be removed smoothly. Alternatively, if internal fixation device 10 is not threaded, internal fixation device 10 can be tapped up and removed using forceps or other device that grips screwdriver 20 or internal fixation device 10, while bone cement fixation tool 60 restrains movement in the direction of pulling out bone cement 1.

Step 5 ([26]): Remove bone cement fixation tool 60 (in practice, along with internal fixation device 10 and screwdriver 20 from step 4 ([25]) and suture incised skin 94 to complete the treatment. When the tip section of bone cement fixation tool 60 and hole 2 are both prism or otherwise shaped, internal fixation device 10 can be removed by simply pulling it out.

FIG. 14 is an explanatory drawing schematically illustrating another example procedure for removing internal fixation device 10.

Initial state ([31]): As in the initial state shown in FIG. 13 ([21]), in the patient's vertebral body 90, bone cement 1 is internally fixed by being supported by internal fixation device 10 inserted through pedicle 91. Hole 2 in predetermined shape is formed in bone cement 1 through through hole 16 in internal fixation device 10.

Step 1 ([32]): Incise and drill subcutaneous tissue from the patient's back body surface, i.e., skin 94, to the affected vertebral body 90, and insert bone cement fixation tool 60 directly into through hole 16 of internal fixation device 10 instead of guide pin 40 shown in [22] of FIG. 13. More specifically, the tip of bone cement fixation tool 60 inserted into the through hole of screwdriver 20 is introduced into through hole 16 of internal fixation device 10 inserted into pedicle 91. In bone cement fixation tool 60, it is more suitable if handle 62 is configured to be detachable. With handle 62 removed, bone cement fixation tool 60 is inserted into through hole 16 of internal fixation device 10 in the same manner as guide pin 40, and screwdriver 20 is guided from the far end of bone cement fixation tool 60 to internal fixation device 10 along the shaft of bone cement fixation tool 60, which then can be connected to the head (connection) 13 of internal fixation device 10.

Step 2 ([33]): Push screwdriver 20 deep along bone cement fixation tool 60 and connect it with head (connection) 13 of internal fixation device 10. Insert bone cement fixation tool 60 more deeply and insert tip section 61 of bone cement fixation tool 60 into hole 2 of bone cement 1. The insertion of screwdriver 20 and bone cement fixation tool 60 may be performed at the same time, either first or second, in any back and forth relationship. Tip section 61 of bone cement fixation tool 60 and hole 2 of bone cement 1 are machined to the same shape and fit together to inhibit rotational movement. The movement in the direction of pulling out bone cement 1 is inhibited by the tip of bone cement fixation tool 60 contacting the bottom of hole 2.

Step 3 ([34]): with tip section 61 of bone cement fixation tool 60 fit into hole 2 of bone cement 1 and inhibited rotation and movement in the direction of pulling out, rotate screwdriver 20 to remove internal fixation device 10 while holding bone cement fixation tool 60 with handle 62 to prevent it from rotating. If the threads 15 on the distal side of internal fixation device 10 tapped pedicle 91 during insertion, then by rotating the device in the reverse direction of insertion, internal fixation device 10 can be given driving force in the removal direction, allowing internal fixation device 10 to be removed smoothly. Alternatively, if internal fixation device 10 is not threaded, internal fixation device 10 can be tapped up and removed using forceps or other device that grips screwdriver 20 or internal fixation device 10, while bone cement fixation tool 60 restrains movement in the direction of pulling out bone cement 1.

Step 4 ([35]): Remove handle 62 from bone cement fixation tool 60 and remove the removed internal fixation device 10 and screwdriver 20.

Step 5 ([36]): Remove bone cement fixation tool 60 and the suture incised skin 94 to complete the treatment. While when tip section 61 of bone cement fixation tool 60 is tightly engaged in hole 2 of bone cement 1, handle 62 is attached for removal, when tip section 61 just fit, bone cement fixation tool 60, internal fixation device 10 and screwdriver 20 may be removed as a single unit instead of removing internal fixation device 10 and screwdriver 20 beforehand in the fourth step ([35]).

Steps 3 ([34]) and 4 ([35]) are the same as steps 4 ([25]) and 5 ([26]) in the example shown in FIG. 13. Bone cement fixation tool 60 is inserted directly into internal fixation device 10 to be removed without using guide pin 40 in the first step, which reduces the number of procedures.

The procedure was described assuming that the tip portion of internal fixation device 10 is provided with threads or grooves and that insertion into and removal from bone cement 1 is done by rotation and counter-rotation of the screw. However, if the tip portion of internal fixation device 10 is not provided with threads or other mechanism, and insertion and removal of internal fixation device 10 into bone cement 1 is done by simple pushing and pulling, this procedure can be applied in the same manner. At that time, bone cement fixation tool 60 is inhibited from moving in the same direction of pulling out as internal fixation device 10 is pulled out, rather than in the direction of rotation of bone cement 1.

### [Embodiment 2]

In Embodiment 1, the solution principle is to inhibit rotation of bone cement 1 by molding a predetermined shaped hole 2 with tip section 51 of bone cement molding tool 50 in bone cement 1, and fitting tip section 61 of bone cement fixation tool 60 of the same shape into hole 2. However, tip section 61 of bone cement fixation tool 60 does not need to have the same shape as tip section 51 of bone cement molding tool 50, it is sufficient if tip section 61 fits into hole 2 formed with tip section 51 of bone cement molding tool 50 to inhibit rotation of bone cement 1.

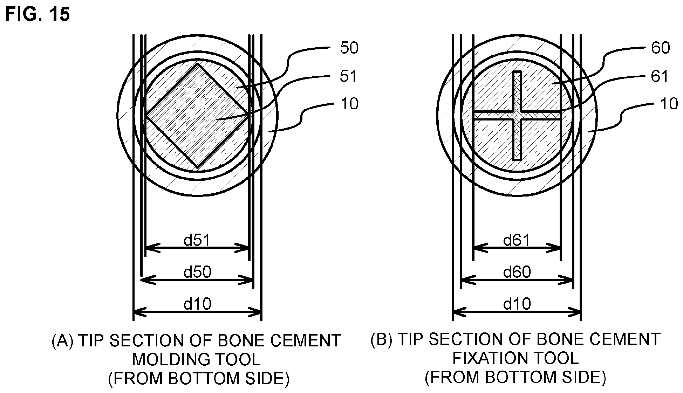

FIG. 15 is an explanatory drawing illustrating the tip sections to show an example configuration of bone cement molding tool 50 and bone cement fixation tool 60 which are examples of the treatment devices of a second embodiment of the present invention, showing bottoms viewed from the proximal ends (bottom side).(a) shows tip section 51 of bone cement molding tool 50. Bone cement molding tool 50 has the same shape as bone cement molding tool 50 of Embodiment 1 shown in FIG. 3, and has a round column shaft and a quadrangular prism-shaped tip section 51, while bone cement fixation tool 60 has a round column shaft and a prism-shaped tip section 61. The diameter d50 of the round column shaft of bone cement molding tool 50 is slightly smaller than the diameter d10 of through hole 16 of bone cement 10, and the quadrangular prism of tip section 51 is formed inside the round column shaft. For example, the diagonal length d51 of a square of the cross section of a regular quadrangular prism is equal to or smaller than d50.

(b) shows tip section 61 of bone cement fixation tool 60. Bone cement fixation tool 60 has a round column shaft and round column tip section 61 with a cross-shaped cross section. The diameter d60 of the round column shaft of bone cement fixation tool 60 is slightly smaller than the diameter d10 of through hole 16 of bone cement 10, and tip section 61 is formed inside the round column shaft. For example, the width d61 of the cross-shaped cross section should be equal to or smaller than d51, and should be large enough to fit approximately tightly into hole 2 formed by tip section 51 of bone cement molding tool 50. Since it is sufficient to fit and prevent bone cement 1 from rotating, there may be some tolerance rather than a snug fit.

While FIG. 15 shows a quadrangular prism and a cross shape for illustration, a single line shape that fits into the diagonals of a quadrangular prism may also be used. Other variations include a hexagonal column for tip section 51 for bone cement molding tool 50, a triangular column for tip section 61 of bone cement fixation tool 60, and a tapered cross-shaped tip.

### [Embodiment 3]

Further variations will be described below. Tip section 51 of bone cement molding tool 50 is round column. For example, the following is case is expected: at the time of implanting internal fixation device 10 into the vertebral body, there is no special bone cement molding tool 50, and a guide pin, a round column bone cement injector, or a cement push-in tool is substituted to form hole 2. Since hole 2 is circular, there are no options for the shape of tip section 61 of bone cement fixation tool 60 that would allow for the inhibition of rotation of bone cement 1 by simply fitting it in.

FIG. 16 is an explanatory drawing illustrating the overviewed appearance of the tip portions to show an example configuration of bone cement molding tool 50 and bone cement fixation tool 60 which are examples of the treatment devices of the third embodiment of the present invention. Tip section 51 of bone cement molding tool 50 is round column, as shown in FIG. 16(a). Tip section 61 of bone cement fixation tool 60 has threads is screwed while being tapped into hole 2 formed by tip section 51 of bone cement molding tool 50, and a rounded tip that abuts the bottom of hole 2 to stop insertion, as shown in FIG. 16(b). More specifically, the circumference of the round column portion of tip section 61 of bone cement fixation tool 60 is smaller than the diameter of hole 2, and the diameter of the perimeter of the thread apexes for tapping is equal to or larger than the diameter of hole 2. This diameter should theoretically be larger than the diameter of hole 2 for tapping. In reality, however, since bone cement 1 is not fully hardened when hole 2 is molded and it is not entirely unlikely that hole 2 will be smaller after bone cement molding tool 50 is removed, it should also be considered that the diameter of the perimeter of the thread apexes for tapping of the tip section 61 of bone cement fixation tool 60 is set to be equal to the diameter of hole 2.

This can provide another bone cement fixation tool 60 that can reduce rotation of bone cement 1. For example, by forming hole 2 in bone cement 1 using guide pin 40 or bone cement injector 80 as an example of a bone cement molding tool 50, and by equipping tip section 61 of bone cement fixation tool 60 with a tapping screw thread of equal or larger outer diameter than the thickness of guide pin 40, bone cement fixation tool 60 is screwed in and the bone cement 1 is fixed. Then, screwdriver 20 is operated to reverse internal fixation device 10 for removal.

FIG. 17 is an explanatory drawing illustrating the overviewed appearance and cross-sectional structure of tip sections of bone cement molding tool 50 and bone cement fixation tool 60 to show another example configuration thereof. In the example shown in FIG. 16, tip section 51 of bone cement molding tool 50 and tip section 61 of bone cement fixation tool 60 are round column, whereas in the example shown in FIG. 17, tip section 51 of bone cement molding tool 50 and tip section 61 of bone cement fixation tool 60 are round column tapered. Round column tapered is a column with sides that are inclined so that the diameter decreases toward the tip, and can be either a conical base or a cone, as illustrated in the figure. The outer circumference of tip section 51 of bone cement molding tool 50 is smooth, and the outer circumference of tip section 61 of bone cement fixation tool 60 has threads for tapping. As shown in (c), hole 2 molded by tip section 51 of bone cement molding tool 50 becomes narrow toward the bottom, so the threads for tapping formed on the outer circumference of tip section 61 of bone cement fixation tool 60 are screwed into the side wall of hole 2, and tip section 61 of the cement fixation instrument 60 fits into hole 2 and stops before tip section 61 reaches the bottom of hole 2. Bone cement fixation tool 60 can reduce forces in the direction of rotation and pulling out of bone cement 1.

In FIG. 17, the respective bottom diameters (the side in contact with the shaft) of tip section 51 of bone cement molding tool 50 and tip section 61 of bone cement fixation tool 60 are depicted as smaller than the shaft, but these diameters may be the same as that of the shaft. However, the diameter of the outer circumference of the threads on tip section 61 of bone cement fixation tool 60 should be smaller than the diameter of through hole 16 of internal fixation device 10.

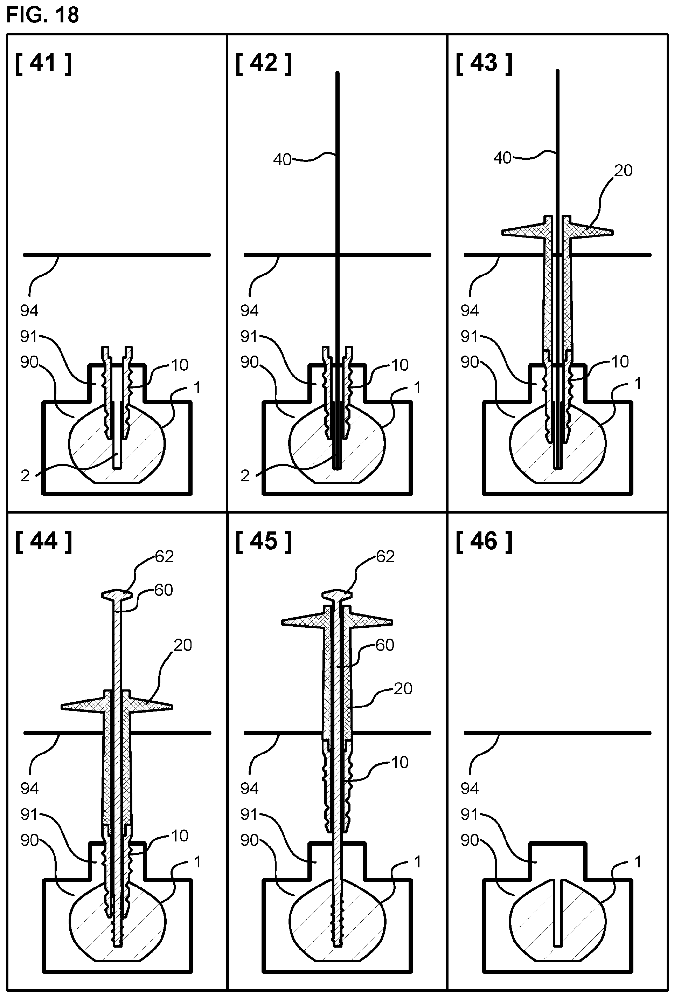

FIG. 18 is an explanatory drawing schematically illustrating an example of the procedure of removing the internal fixation device 10 in treatment using the treatment device of the third embodiment.

Initial state ([41]): In the patient's vertebral body 90, bone cement 1 is internally fixed by being supported by internal fixation device 10 inserted through pedicle 91. Hole 2 in predetermined shape is formed in bone cement 1 through through hole 16 in internal fixation device 10. In FIG. 18, hole 2 assumes a cylindrical hole formed by round column, but as mentioned above, the same is true for round column tapered (conical base or cone) instead of round column.

Step 1 ([42]): Incise and drill subcutaneous tissue from the patient's back body surface, i.e., skin 94, to the affected vertebral body 90, and introduce guide pin 40 in through hole 16 of internal fixation device 10 inserted in pedicle 91. Although FIG. 18[42] illustrates a state in which guide pin 40 is introduced to reach hole 2 formed in bone cement 1 in vertebral body 90, guide pin 40 only needs to reach through hole 16 of internal fixation device 10.

Step 2 ([43]): Guide screwdriver 20 to internal fixation device 10 by aligning the through hole of screwdriver 20 with the inserted guide pin 40, and connect the tip of screwdriver 20 by fitting it into head (connection) 13 of internal fixation device 10.

Step 3 ([44]): Insert bone cement fixation tool 60 into the through hole of screwdriver 20 and through hole 16 of internal fixation device 10, which are in communication when screwdriver 20 and internal fixation device 10 are connected, and guide bone cement into vertebral body 90, inserting tip section 61 into hole 2 of bone cement 1. Tip section 61 of bone cement fixation tool 60 has threads for tapping, so it advances while tapping hole 2 in bone cement 1 and stops when tip section 61 reaches the bottom of hole 2. Then, tip section 61 proceeds tapping while holding down the screwdriver 20 to inhibit rotation of bone cement 1. Tapping creates a force to pull internal fixation device 10 out in a direction opposite to the force of rotation of bone cement 1, creating a gap between internal fixation device 10 and bone cement 1, allowing removal of internal fixation device 10.

Step 4 ([45]): with tip section 61 of bone cement fixation tool 60 fit into hole 2 of bone cement 1 and inhibited rotation, rotate screwdriver 20 to remove internal fixation device 10 while holding bone cement fixation tool 60 with handle 62 to prevent it from rotating. If the threads 15 on the distal side of internal fixation device 10 tapped pedicle 91 during insertion, then by rotating the device in the reverse direction of insertion, internal fixation device 10 can be given driving force in the removal direction, allowing internal fixation device 10 to be removed smoothly. For example, if bone cement fixation tool 60 is rotated clockwise when advancing toward the bottom of hole 2, internal fixation device 10 is removed by rotating screwdriver 20 counterclockwise while maintaining that clockwise force. Even if bone cement 1 is to be rotated counterclockwise with the force of rotating screwdriver 20 counterclockwise, the rotation of bone cement 1 can be reduced by the force of rotating bone cement fixation tool 60 clockwise.

Step 5 ([46]): Remove bone cement fixation tool 60 and the suture incised skin 94 to complete the treatment. Tip section 61 of bone cement fixation tool 60 can be removed by rotating it in the opposite direction of insertion. At this time, no force can be applied to inhibit rotation of bone cement 1; however, since the insertion of bone cement fixation tool 60 is a tapping of bone cement 1 that has completely hardened after a long period of time after hardening, bone cement 1 can be pulled out with weak force without rotation of bone cement 1. Furthermore, by loosening bone cement fixation tool 60 immediately after internal fixation device 10 is slightly loosened from its joint to bone cement 1, it can be prevented that bone cement fixation tool 60 would be getting stuck and not being able to be removed.

In the unlikely event that bone cement 1 is easily rotated away from the surrounding body tissue, two different types of bone cement fixation tools can be used: bone cement fixation tool 60 with tip section 61 with a relatively large outer diameter thread to form a tapping groove and bone cement fixation tool 60 with tip section 61 with a smaller outer diameter thread. Bone cement fixation tool 60 with a larger outer diameter thread forms the tapping groove, and then bone cement fixation tool 60 with a smaller outer diameter thread is inserted along the formed tapping groove to the bottom of hole 2. Bone cement fixation tool 60 with threads with a smaller outer diameter is used to reduce rotation of bone cement 1 when internal fixation device 10 is to be removed. Since this bone cement fixation tool 60 has threads with a smaller outer diameter and is just inserted along the preformed tapping groove, it can be pulled out with weak force by reversing the rotation.

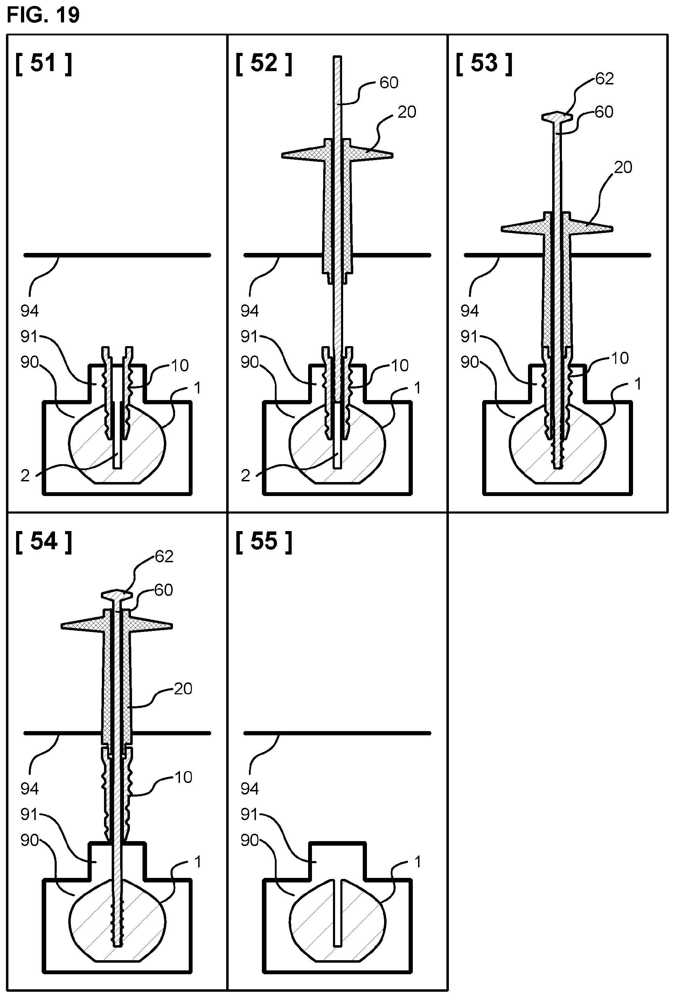

FIG. 19 is an explanatory drawing schematically illustrating another example of the procedure of removing the internal fixation device 10 in treatment using the treatment device of the third embodiment.

Initial state ([51]): As in FIG. 18 ([41]), in the patient's vertebral body 90, bone cement 1 is internally fixed by being supported by internal fixation device 10 inserted through pedicle 91. Hole 2 in predetermined shape is formed in bone cement 1 through through hole 16 in internal fixation device 10. In FIG. 19, hole 2 assumes a cylindrical hole formed by a column, but the same is true for round column tapered instead of round column.

Step 1 ([52]): Incise and drill subcutaneous tissue from the patient's back body surface, i.e., skin 94, to the affected vertebral body 90, and insert bone cement fixation tool 60, instead of guide pin 40 in FIG. 18[42], into through hole 16 of internal fixation device 10 that has been inserted into pedicle 91. With handle 62 removed from bone cement fixation tool 60, bone cement fixation tool 60 may be first inserted into through hole 16 of internal fixation device 10, or screwdriver 20 may be inserted beforehand from the tip side of bone cement fixation tool 60 with handle 62 attached, and then tip section 61 of the bone cement fixation tool 60 may be inserted into through hole 16 of internal fixation device 10 while being held it by hand.

Step 2 ([53]): Attach handle 62 to bone cement fixation tool 60 and screw tip section 61 into hole 2, guide screwdriver 20 to internal fixation device 10 by aligning the through hole of screwdriver 20 with inserted bone cement fixation tool 60, and connect the tip of screwdriver 20 by fitting it into the head (connection) of internal fixation device 10 ) 13 The screwing of tip section 61 of bone cement fixation tool 60 into hole 2 may be done after the connection of screwdriver 20 and internal fixation device 10.

Step 3 ([54]): with tip section 61 of bone cement fixation tool 60 fit into hole 2 of bone cement 1 and inhibited rotation, rotate screwdriver 20 to remove internal fixation device 10 while holding bone cement fixation tool 60 with handle 62 to prevent it from rotating. If the threads 15 on the distal side of internal fixation device 10 tapped pedicle 91 during insertion, then by rotating the device in the reverse direction of insertion, internal fixation device 10 can be given driving force in the removal direction, allowing internal fixation device 10 to be removed smoothly. For example, if bone cement fixation tool 60 is rotated clockwise when advancing toward the bottom of hole 2, internal fixation device 10 is removed by rotating screwdriver 20 counterclockwise while maintaining that clockwise force. Even if bone cement 1 is to be rotated counterclockwise with the force of rotating screwdriver 20 counterclockwise, the rotation of bone cement 1 can be reduced by the force of rotating bone cement fixation tool 60 clockwise.

Step 4 ([55]): Remove bone cement fixation tool 60 and the suture incised skin 94 to complete the treatment.

As described above, the removal procedure can be simplified by using bone cement fixation tool 60 in place of guide pin 40 in FIG. 18. It is more suitable if bone cement fixation tool 60 is configured to allow handle 62 to be attached to and detached from bone cement fixation tool 60.

### [Embodiment 4]

FIG. 20 is an explanatory drawing schematically illustrating example configurations of bone cement molding tool 50 and screwdriver 20 according to the treatment device of fourth and fifth embodiments of the present invention.

The fourth embodiment is a variation from the third embodiment described in FIG. 16, citing FIG. 16. In the fourth embodiment, bone cement fixation tool 60 as well as bone cement molding tool 50 have threads that tap the side wall of hole 2 and screwed thereinto. This allows the grooves to be tapped in advance on the side wall of hole 2. When internal fixation device 10 needs to be removed, the threads for tapping of bone cement fixation tool 60 are screwed along the aforementioned groove preformed in hole 2, allowing the internal fixation device to be screwed in and pulled out with little force and facilitating removal of the bone cement fixation tool after the internal fixation device is removed.

Although FIG. 20 also depicts the fifth embodiment described below, these embodiments need not be implemented in combination.

### [Embodiment 5]

In treatment device 100 of Embodiment 5, bone cement molding tool 50 may be configured to be inserted into the through hole of screwdriver 20 that has been connected to internal fixation device 10, protruding a predetermined length (e.g., about 1 cm) from the tip of internal fixation device 10, as illustrated in FIG. 20. This allows bone cement molding tool 50 to be inserted to form the hole while the screwdriver 20 and internal fixation device 10 are connected together, resulting in the reduced the number of procedural steps as described below.

It is even more suitable if bone cement molding tool 50 is configured to be detachable from screwdriver 20. This is because molding of hole 2 and the insertion of internal fixation device 10 into bone cement 1 can be performed selectively: simultaneously or separately.

Each of bone cement molding tool 50 and screwdriver 20 can be configured to, for example, be provided with a screw structure on the distal end that is screwed for connection and integration. Other connection methods may be employed. For example, a connector structure that it cannot be disconnected without a strong pull, or a latch structure that retains the connection may be employed. This improves operability of treatment device 100 when molding of hole 2 and inserting of internal fixation device 10 into bone cement 1 are performed at the same time. This is because, as illustrated, handle 27 of screwdriver 20 and handle 52 of bone cement molding tool 50 can be integrated and configured for easy one-handed gripping.

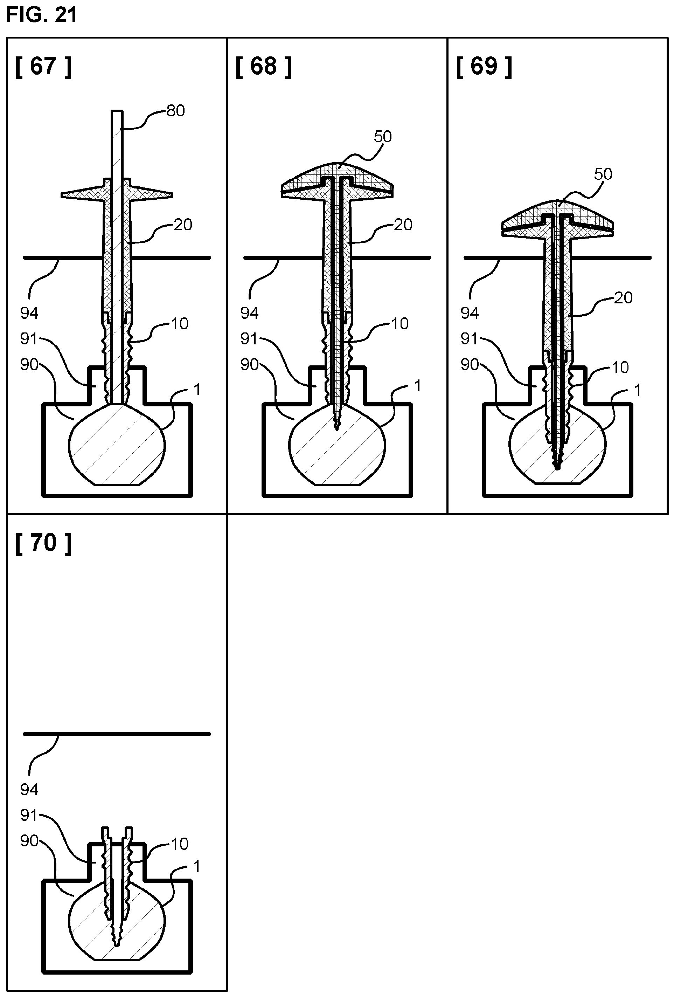

FIG. 21 is an explanatory drawing schematically illustrating the second half of the treatment procedure of implanting internal fixation device 10 in treatment using treatment device 100 according to the fifth embodiment. Since the first half of the procedure for implanting internal fixation device 10 is similar to steps 1 ([1]) through 6 ([6]), described citing FIG. 10, description thereof will be omitted. As in FIGS. 10 and 11, FIG. 21 also schematically shows a cross-section of the patient's affected area from the outer skin 94 through pedicle 91 to vertebral body 90, with the upper side of the paper as distal side and the lower side as proximal side. The scale in the direction of the central axis (vertical direction on the paper) is compressed, and the direction along the patient's body surface (horizontal direction on the paper) is emphasized. Since pedicles 91 are on each side of the vertebral bodies 90, the same procedure is performed from the left and right for one vertebral body 90, FIG. 21 shows one of them.

Step 7 ([67]): Pull balloon catheter 70 out of the through holes of screwdriver 20 and internal fixation device 10 and insert cement injector 80 to inject bone cement 1 into vertebral body 90.

Step 8 ([68]): Pull cement injector 80 out through the through holes of screwdriver 20 and internal fixation device 10, insert bone cement molding tool 50 through the through hole of screwdriver 20, and cause tip section 51 to protrude from the tip of internal fixation device 10 into bone cement 10. Although FIG. 21 is drawn assuming bone cement molding tool 50 illustrated in FIG. 20, this form of bone cement molding tool 50 is not limiting; tip section 51 may be, for example, round column or round column tapered (conical or conical), with or without threads for tapping on the outer surface.

Step 9 ([69]): At the same time as screwing internal fixation device 10 into the filled bone cement 1 using screwdriver 20, insert bone cement molding tool 50 into bone cement 1 to mold hole 2. As illustrated in FIG. 21, bone cement molding tool 50 is more suitable if it is integrated with screwdriver 20 by a screw structure at the far end. However, this is not limiting.

Step 10 ([70]): Pull screwdriver 20 out and suture incised skin 94 to complete the treatment.

As described above, compared to the procedure described in FIG. 11, the procedure can be shortened to the extent that the insertion of internal fixation device 10 and the molding of hole 2 with bone cement molding tool 50 can be performed at the same time.

### [Embodiment 6]

In the first to fifth embodiments described above, bone cement fixation tool 60 may be provided with a through hole through which the guide pin can pass. This allows bone cement fixation tool 60 to be guided into hole 2 of the affected bone cement 1 along guide pin 40 that has been inserted into the affected area beforehand.

FIG. 22 is an explanatory drawing schematically illustrating an example of the procedure of removing the internal fixation device 10 in treatment using the treatment device of a sixth embodiment according to the present invention.

Initial state ([71]): In the patient's vertebral body 90, bone cement 1 is internally fixed by being supported by internal fixation device 10 inserted through pedicle 91. Hole 2 in predetermined shape is formed in bone cement 1 through through hole 16 in internal fixation device 10.

Step 1 ([72]): Incise and drill subcutaneous tissue from the patient's back body surface, i.e., skin 94, to the affected vertebral body 90, and insert guide pin 40 in through hole 16 of internal fixation device 10 inserted in pedicle 91, and the tip is allowed to reach hole 2 formed in bone cement 1 inside vertebral body 90.

Step 2 ([73]): Guide bone cement fixation tool 60, together with screwdriver 20, in the direction of hole 2 formed in bone cement 1 by aligning it with guide pin 40.

Step 3 ([74]): insert bone cement fixation tool 60 into hole 2, connect screwdriver 20 with internal fixation device 10 and head 13, and remove guide pin 40.

Step 4 ([75]): with tip section 61 of bone cement fixation tool 60 fit into hole 2 of bone cement 1 and inhibited rotation and/or movement in the direction of pulling out, rotate screwdriver 20 to remove internal fixation device 10 while holding bone cement fixation tool 60 with handle 62 to prevent it from rotating and pulling out. As in the other embodiments described above, if the threads 15 on the distal side of internal fixation device 10 tapped pedicle 91 during insertion, then by rotating the device in the reverse direction of insertion, internal fixation device 10 can be given driving force in the removal direction, allowing internal fixation device 10 to be removed smoothly. Alternatively, if internal fixation device 10 is not threaded, internal fixation device 10 can be tapped up and removed using forceps or other device that grips screwdriver 20 or internal fixation device 10, while bone cement fixation tool 60 restrains movement in the direction of pulling out bone cement 1.

Step 5 ([76]): Remove bone cement fixation tool 60 and the suture incised skin 94 to complete the treatment.

As described above, by providing a through hole in bone cement fixation tool 60 through which a guide pin can pass, bone cement fixation tool 60 can be easily and accurately guided through hole 2 formed by bone cement molding tool 50, allowing the procedure for removing internal fixation device 10 to proceed smoothly.

While the present invention made by the present inventor has been described in detail with respect to the embodiments thereof, the present invention is not limited to these embodiments. Various changes may be made without departing from the spirit and scope of the present invention.

### Industrial Applicability

The present invention relates to a round column internal fixation device for medical use, and is particularly suitable for use in internal fixation surgery of affected vertebral body.

### EXPLANATION OF SIGN

1 Bone cement
2 hole
10 Internal fixation device
12 Shaft
13 Head (connection with screwdriver)
14 Groove
15 Ridge
16 Through hole
17 Tapered Section
20 Screwdriver
25 Connection with inner cylinder
26 External thread
27 Handle of screwdriver
30 Inner cylinder
31 Tongue piece of inner cylinder
32 Tip section of inner cylinder
33 Notch
34 Thread at tip section of inner cylinder
35 Connection with screwdriver
36 Internal thread
37 Handle of inner cylinder
40 Guide pin
50 Bone cement molding tool
51 Tip section of bone cement molding tool
52 Handle
53 Connection
54 Ridge
60 Bone cement fixation tool
61 Tip section of bone cement fixation tool
62 Handle
64 Through hole
70 Balloon catheter
71 Balloon
80 Cement injector
90 Vertebral body
91 pedicle
92 Vertebral arch
93 Spinal canal
94 Skin
100 Treatment device

## Claims

1. A treatment device comprising a bone cement molding tool and a bone cement fixation tool, which are inserted into an internal fixation device that supports and internally fixes bone cement filled into a vertebral body from a pedicle, wherein
the internal fixation device has a through hole in the direction of a central axis from a head to a tip and can be inserted into and pulled out of the bone cement along the central axis,
the bone cement molding tool is inserted into the bone cement through the through hole in the internal fixation device to mold a hole in a predetermined shape,
the bone cement fixation tool has a tip section to be inserted into the hole and is inserted into bone cement in which the hole has been formed through the through hole in the internal fixation device to inhibit rotation around the central axis and/or movement in the pull-out direction of the bone cement.

2. The treatment device according to claim 1, wherein
a tip section of the bone cement molding tool is cylindrical or round column tapered with a decreasing diameter toward the tip, and
the bone cement fixation tool has threads that tap a side wall of the hole and screwed thereinto.

3. The treatment device according to claim 2, wherein
the bone cement molding tool has threads that tap a side wall of the hole and screwed thereinto.

4. The treatment device according to claim 2, wherein
the treatment device further comprises a screwdriver,
the internal fixation device being connectable to the screwdriver at the head, and
the screwdriver has, when connected to the internal fixation device, a through hole that communicates with a through hole of the internal fixation device and through which the respective tip portions of the bone cement molding tool and the bone cement fixation tool can pass.

5. The treatment device according to claim 4, wherein
the bone cement molding tool is inserted into the through hole of the screwdriver connected to the internal fixation device and protrudes a predetermined length from the tip of the internal fixation device.

6. The treatment device according to claim 5, wherein
the bone cement molding tool is detachably connected to the screwdriver.

7. The treatment device according to claim 6, wherein
the screwdriver has a screw structure at the distal end that can be connected to the bone cement molding tool.

8. The treatment device according to claim 2, wherein
the bone cement fixation tool has a structure in which a handle portion can be detached from a shaft portion.

9. The treatment device according to any one of claims 2 to 8, wherein
the treatment device further includes a guide pin, and the bone cement fixation tool has a through hole through which the guide pin can pass.

10. The treatment device according to claim 1, wherein
a tip section of the bone cement molding tool is prism shaped or prism tapered with a decreasing diameter toward the tip, and
the bone cement fixation tool has a tip shape that is able to contact a side wall of the hole and inhibit rotation of the bone cement.

11. The treatment device according to claim 10, wherein
the bone cement fixation tool has a structure in which a handle portion can be detached from a shaft portion.

12. The treatment device according to claim 10 or 11, wherein
the treatment device further comprises a screwdriver,
the internal fixation device being connectable to the screwdriver at the head, and
the screwdriver, when connected to the internal fixation device, communicating with the through hole of the internal fixation device and having a through hole through which tip portions of the bone cement molding tool and the bone cement fixture tool can pass.

13. The treatment device according to any one of claims 10 to 12, wherein
the treatment device further includes a guide pin, and the bone cement fixation tool has a through hole through which the guide pin can pass.
